# EUROPEAN PATENT APPLICATION

(11) **EP 4 520 819 A1**
(43) Date of publication of application: **12.03.2025**
(21) Application number: 23799262.3
(22) Date of filing: 04.05.2023
(51) Int. Cl.: C12N 9/02, C12N 15/53, C12P 17/10

(54) **CHEMICAL-ENZYME COUPLING METHOD FOR SYNTHESIZING ERGOTHIONEINE**

(30) Priority: 03.05.2022 CN 202210477190
(71) Applicant: Shanghai Institute of Organic Chemistry, Chinese Academy of Sciences, Shanghai 200032 (CN)
(72) Inventor: LIN, Liang, Shanghai 200032 (CN); LV, Long, Shanghai 200032 (CN); JIANG, Yan, Shanghai 200032 (CN); WU, Yaming, Shanghai 200032 (CN); SONG, Shucheng, Shanghai 200032 (CN); SUN, Lili, Shanghai 200032 (CN); DING, Xiaoran, Shanghai 200032 (CN)
(74) Representative: Mewburn Ellis LLP
(86) International application number: PCT/CN2023/092114
(87) International publication number: WO 2023/213276

(57) **Abstract**

Provided is a chemical-enzyme coupling method for synthesizing ergothioneine. The method comprises the steps of chemically synthesizing histidine betaine, and synthesizing ergothioneine from the histidine betaine by using Schizosaccharomyces pombe ergothioneine synthetases SPEGT1-tr M10 and SPEGT2 M3 after directed evolution.

## Description

### Technology Field

The invention relates to the field of synthesis technology. In particular, the invention provides a large-scale chemical synthesis method for the L-histidine betaine, and synthesizing ergothioneine from the L-histidine betaine by using *Schizosaccharomyces pombe* ergothioneine synthetases SPEGT1-tr M10 and SPEGT2 M3 after directed evolution

### Background Technology

Ergothioneine (EGT) is an amino acid of a special sulfurine histidine betaine. Its unique oxidation and reduction characteristics make it one of the best natural antioxidants. EGT is mainly synthesized by microorganisms such as fungi and line bacteria, which has a unique physiological effect on plants and animals. EGT is equivalent to the rare vitamins of animals, and have a good resistance to oxidative stress. Therefore, EGT can be used as antioxidants and potential nutritional foods, and has great application prospects in the food, cosmetics and medicine industries.

At present, it is difficult to obtain the correct chirality of EGT by chemical synthesis method, and the production capacity of biological extraction is insufficient. On the one hand, although EGT synthetases EGT1 and EGT2 of fungi such as Neurospora crassa and Pleuropleoides have been reported, they have been heterogeneously expressed in Escherichia coli for the synthesis of EGT in vitro. However, due to the low yield of synthetic EGT in the prior art, it is far from reaching the potential of industrial production amplification. On the other hand, the synthesis of EGT by fermentation without adding amino acids can reach a maximum yield of 1.5g per liter, but the production cost is still high and the production cycle is long.

To sum up, there is still a lack of an efficient method to produce EGT in the field.

### Summary

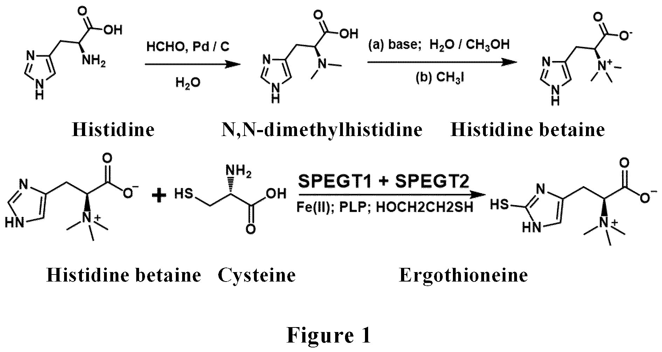

In order to overcome the shortcomings and deficiencies of the prior art, the invention combines the advantages of both chemical synthesis and biosynthesis, aiming to provide an efficient synthesis system of chemical enzyme coupling of EGT(as shown in Figure 1). L-histidine betaine is synthesized by the use of palladium-carbon catalyzed formaldehyde reduction amination and potassium iodide nucleophilic substitution, followed by using EGT synthetase SPEGT1 (Uniprot ID: O94632) and SPEGT2 (Uniprot ID: 094431) mutant engineering enzymes SPEGT1-tr M10 and SPEGT2 M3 by *Schizosaccharomyces pombe* to synthesize EGT from L-histidine betaine and cysteine.

In addition, the invention also provides a screening method for EGT1 and EGT2, and a 50mL reaction system of high concentration substrate. The invention provides a condition optimization method for EGT enzyme catalysis, determines the combination of EGT synthetase EGT1 and EGT2 based on *Schizosaccharomyces pombe,* and optimizes the dosage of Fe(II), pyridoxal phosphate (PLP) and sulfur/phosphorus containing reducing agent in the enzyme catalytic system.

The first aspect of the invention provides a combination of *Schizosaccharomyces pombe* ergothioneine synthetases, wherein the combination comprises: SPEGT1-tr M10, whose amino acid sequence is shown in SEQ ID NO: 7; and SPEGT2 M3, whose amino acid sequence is shown in SEQ ID NO: 9.

In another preferred embodiment, the nucleotide sequence of the gene encoding the SPEGT1-tr M10 synthase (*spegt1-tr M10*) is shown in SEQ ID NO: 8.

In another preferred embodiment, the nucleotide sequence of the gene encoding the synthase (*spegt2 M3*) is shown in SEQ ID NO: 10.

The second aspect of the invention provides an ergothionein synthetase, wherein the methylation domain of the synthetases is truncated, and the synthetase is selected from the group consisting of: SPEGT1-tr, SPEGT1-tr M10, SOcEGT1-tr, SCrEGT1-tr, SOsEGT1-tr, SJEGT1-tr, TDEGT1-tr, PLEGT1-tr, SCoEGT1-tr, TCEGT1-tr, and NCEGT1-tr, whose amino acid sequence is shown in SEQ ID NO: 5, 7, 11, 13, 15, 17, 19, 21, 23, 25, 27.

Another aspect of the invention provides a *Schizosaccharomyces pombe* ergothioneine synthetase SPEGT1-tr, whose amino acid sequence is shown in SEQ ID NO: 5.

In another preferred embodiment, the nucleotide sequence of the gene encoding the synthetase (*spegt1 tr*) is shown in SEQ ID NO: 6.

In another preferred embodiment, the invention provides a *Schizosaccharomyces pombe* ergothioneine synthetase SPEGT1, whose amino acid sequence is shown in SEQ ID NO: 1.

In another preferred embodiment, the nucleotide sequence of the gene encoding the synthetase (*spegt1*) is shown in SEQ ID NO: 2.

In another preferred embodiment, the invention provides a *Schizosaccharomyces pombe* ergothioneine synthetase SPEGT2, whose amino acid sequence is shown in SEQ ID NO: 3.

In another preferred embodiment, the nucleotide sequence of the gene encoding the synthetase (*spegt2*) is shown in SEQ ID NO: 4.

Another aspect of the invention provides a series of ergothioneine synthetase EGT1-tr of other strains, whose amino acid sequence is shown in SEQ ID NO: 11, 13, 15, 17, 19, 21, 23, 25, 27.

Another aspect of the invention provides a series of ergothioneine synthetase EGT2 of other strains, whose amino acid sequence is shown in SEQ ID NO: 29, 31, 33, 35, 37, 39, 41, 43, 45.

The third aspect of the invention provides an expression vector for ergothioneine synthetase, wherein the expression vector is used to express ergothioneine synthetase as described in any one of the first to second aspects of the invention.

In another preferred embodiment, the expression vector expresses a synthetic enzyme as described in the first or second aspect of the invention.

The fourth aspect of the invention provides an expression kit for expressing ergothioneine synthetase as described in any one of the first to second aspects of the invention.

The fifth aspect of the invention provides a recombinant strain, wherein the recombinant strain comprises an expression vector as described in the third aspect of the invention, or its genome integrates a polynucleotide sequence encoding the synthetase as described in any one of the first to second aspects of the invention.

In another preferred embodiment, the recombinant strain genome integrates a nucleotide sequence encoding the synthetases as described in the first and/or second aspects of the invention.

The sixth aspect of the invention provides a chemical enzyme coupling system for the synthesis of ergothioneine, wherein the chemical catalytic system comprises: palladium-carbon catalyzed a reduction amination dimethylation of L-histidine with formaldehyde, and undergoes a nucleophilic substitution with iodomethane to synthesize trimethylated quaternary ammonium histidine betaine;
the catalytic enzyme system comprises a combination of ergothioneine synthetase as described in the first to second aspects of the invention.

The seventh aspect of the invention provides a method for synthesizing ergothioneine, comprising the following steps:

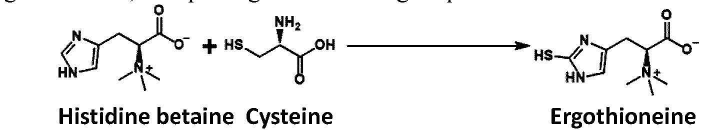

In the presence of the combination of ergothioneine synthatase described in the first to second aspects of the invention, or in the presence of the recombinant strain described in the fifth aspect of the invention, reacting histidine betaine with cysteine to obtain the ergothioneine.

In another preferred embodiment, the method further comprises synthesizing histidine betaine through chemical synthesis steps; preferably, the chemical synthesis step comprises: 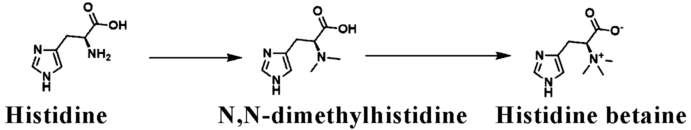
(1) under palladium-carbon catalysis, L-histidine undergoes reductive amination with formaldehyde to obtain dimethyl histidine;
(2) dimethyl histidine undergoes nucleophilic substitution with iodomethane to synthesis a trimethyl substituted quaternary ammonium, histidine betaine.

In another preferred embodiment, the enzyme catalytic step is carried out in the presence of Fe²⁺, pyridoxal phosphate, and a reducing agent; wherein the reducing agent is thiol reducing agent or a phosphine reducing agent used for disulfide bond reduction.

In another preferred embodiment, the thiol reducing agent is selected from the group consisting of: beta mercaptoethanol, cysteamine, DTT, DTE, GSH, DTBA, DMH, Meso-DTA, BMS and BMMP, and/or
the phosphine reducing agent is selected from the group consisting of: THMP, TCEP and THPP.

In another preferred embodiment, the enzyme catalytic step is carried out in the presence of Fe²⁺, pyridoxal phosphate, and beta mercaptoethanol.

In another preferred embodiment, the reaction system of the enzyme catalyzed step further comprises one or more features selected from the group consisting of:
(1) the Fe²⁺ content is 0-40 mM, preferably 10-40 mM;
(2) the content of pyridoxal phosphate is 0-4 mM, preferably 1-4 mM;
(3) the reducing agent content is 50-200 mM;
(4) the pH range is 4.5-10, preferably 7-9.

In another preferred embodiment, the enzyme catalytic step is carried out at 10-45 °C, and preferably, the enzyme catalytic step is carried out at 20-30 °C.

It should be understood that within the scope of the invention, the above-mentioned technical features of the invention and the specific technical features described below (such as examples) can be combined with each other to form new or preferred technical solutions. Due to space limitations, it will not be repeated here.

### Brief Description of the Drawing

Figure 1 shows the chemical enzyme coupling method for the synthesis of ergothioneine.
Figure 2 shows the scheme of the E. coli expression plasmids pET-28a-SpEgt1, pET-28a-SpEgt1-tr, and pET-28a-SpEgt2, which express the *Schizosaccharomyces pombe* ergothioneine synthetases SPEGT1, SPEGT1-tr after removing the catalytic methylation domain, and the SPEGT2 encoding gene;
Figure 3 shows the agarose gel electrophoresis image (bands 1 and 3) of the plasmid pET-28a-SpEgt1-tr using the random mutation kit for PCR in directed evolution and the agarose gel electrophoresis (bands 2 and 4) of the complementary vector part for PCR. It also includes the agarose gel electrophoresis image (bands 5 and 7) of the plasmid pET-28a-SpEgt2 using the random mutation kit for PCR in directed evolution and the agarose gel electrophoresis (bands 6 and 8) of the complementary vector part for PCR.
Figure 4 shows the SDS-PAGE images of *Schizosaccharomyces pombe* ergothioneine synthetases SPEGT1, SPEGT1-tr after removing the catalytic methylation domain, SPEGT1-tr M10 after directed evolution, SPEGT2 and SPEGT2 M3 after directed evolution expressed in E. coli.
Figure 5 shows the HPLC identification image of the standard substance of ergothioneine, as well as the HPLC identification image of the compound mixture after adding the product ergothioneine and the internal standard p-aminobenzoic acid to the reaction system without enzymes.
Figure 6 shows the HPLC identification image of the synthesis of ergothioneine using *Schizosaccharomyces pombe* ergothioneine synthetases SPEGT1-tr and SPEGT2 under 10 mM histidine betaine conditions, as well as the HPLC identification image of the synthesis of ergothioneine using directed evolution SPEGT1-tr M10 and SPEGT2 M3 under 10 mM histidine betaine conditions.
Figure 7 shows the HPLC identification images of the synthesis of ergothioneine using *Schizosaccharomyces pombe* ergothioneine synthetases SPEGT1-tr and SPEGT2 under 100 mM histidine betaine conditions, as well as the HPLC identification image of the synthesis of ergothioneine using directed evolution SPEGT1-tr M10 and SPEGT2 M3 under 100 mM histidine betaine conditions.

### Detailed Description of the Invention

The inventor has obtained a highly efficient chemical enzyme coupling synthesis strategy for ergothioneine through long-term and in-depth research. Among them, a large-scale chemical synthesis method for L-histidine betaine and ergothioneine synthetases based on *Schizosaccharomyces pombe* were mainly obtained. The ergothioneine synthetase removes the catalytic methylation domain on the basis of existing SPEGT1 synthetase, and underwent directional evolution to obtain SPEGT1-tr M10, and directed evolution to obtain SPEGT2 M3 on the basis of SPEGT2 synthetase. The obtained SPEGT1-tr M10 synthetase showed an increased yield in the fermentation expression of genetically engineered bacteria compared to the existing SPEGT1 synthetase, and its efficiency in synthesizing ergothioneine was significantly improved. Based on the above findings, the inventor has completed the invention.

### The Ergothioneine Synthetases from Schizosaccharomyces pombe

The invention provides an application of ergothioneine synthetases SPEGT1 and SPEGT2 form *Schizosaccharomyces pombe.*

The amino acid sequence of the *Schizosaccharomyces pombe* ergothioneine synthetase SPEGT1 is shown in SEQ ID NO: 1:

MTEIENIGALEVLFSPESIEQSLKRCQLPSTLLYDEKGLRLFDEITNLKEYYLYE
SELDILKKFSDSIANQLLSPDLPNTVIELGCGNMRKTKLLLDAFEKKGCDVHFYALD
LNEAELQKGLQELRQTTNYQHVKVSGICGCFERLLQCLDRFRSEPNSRISMLYLGAS
IGNFDRKSAASFLRSFASRLNIHDNLLISFDHRNKAELVQLAYDDPYRITEKFEKNIL
ASVNAVFGENLFDENDWEYKSVYDEDLGVHRAYLQAKNEVTVIKGPMFFQFKPSH
LILIEESWKNSDQECRQIIEKGDFKLVSKYESTIADYSTYVITKQFPAMLQLPLQPCPS
LAEWDALRKVWLFITNKLLNKDNMYTAWIPLRHPPIFYIGHVPVFNDIYLTKIVKN
KATANKKHFWEWFQRGIDPDIEDPSKCHWHSEVPESWPSPDQLREYEKESWEYHIV
KLCKAMDELSTSEKRILWLCYEHVAMHVETTLYIYVQSFQNANQTVSICGSLPEPA
EKLTKAPLWVNVPETEIAVGMPLTTQYTSVGSNLQSSDLSAHENTDELFYFAWDNE
KPMRKKLVSSFSIANRPISNGEYLDFINKKSKTERVYPKQWAEIDGTLYIRTMYGLL
PLDDYLGWPVMTSYDDLNNYASSQGCRLPTEDELNCFYDRVLERTDEPYVSTEGK
ATGFQQLHPLALSDNSSNQIFTGAWEWTSTVLEKHEDFEPEELYPDYTRDFFDGKH
NVVLGGSFATATRISNRRSFRNFYQAGYKYAWIGARLVKN*;

The nucleotide sequence of its encoding gene (*spect1*) is shown in SEQ ID NO: 2:

atgaccgaaattgagaacatcggtgccctggaagtgctgttttctccggaaagtattgaacagagtctgaaacgttgccag
ctgccgagtaccctgctgtatgatgagaaaggtctgcgcctgtttgatgaaatcaccaatctgaaagaatactacctgtatgaaagc
gaactggatattctgaagaaattcagcgatagcattgccaatcagctgctgagtccggatctgccgaataccgttattgaactgggc
tgtggcaatatgcgtaagactaaactgctgctggatgcctttgagaagaaaggctgcgatgttcatttctatgccctggatctgaatg
aagccgaactgcagaaaggtctgcaggaactgcgccagaccaactatcagcatgttaaagtgagtggtatttgcggttgtttc
gaacgcctgctgcagtgtctggatcgtttccgtagcgaaccgaatagtcgtattagcatgctgtatctgggtgcaagtattggtaact
tcgatcgcaaatcagccgcaagtttcctgcgtagtttcgcaagccgtctgaacatccatgataatctgctgattagtttcgatcatcgc
aacaaagccgaactggttcagctggcctatgatgatccgtatcgcattaccgagaaattcgagaagaacattctggccagcgttaa
tgcagtgtttggcgagaatctgtttgatgagaatgattgggaatacaaatccgtttatgacgaagatctgggtgtgcatcgcgcatat
ctgcaggcaaagaatgaagttaccgtgatcaaaggtccgatgttctttcagttcaaaccgagtcatctgattctgattgaagaaagtt
ggaagaatagtgatcaggaatgccgtcagatcatcgagaaaggcgatttcaaactggttagcaaatacgaaagtaccattgccga
ttacagcacctatgtgattaccaaacagtttccagccatgctgcagctgccgctgcagccttgtccgagcctggcagaatgggatg
ccctgcgcaaagtgtggctgttcattaccaacaaactgctgaataaggacaatatgtacaccgcctggattccgctgcgtcatccac
cgatcttctacattggccatgtgccggtgttcaacgatatctacctgaccaagattgtgaagaataaggcaaccgccaataagaaac
atttctgggaatggtttcagcgtggtattgatccggatattgaagatccgagcaaatgccattggcatagtgaagttccggaaagttg
gccgtctccggatcagctgcgtgaatatgagaaagaaagttgggaatatcatatcgtgaaactgtgtaaagcaatggatgaactga
gtaccagtgagaaacgtattctgtggctgtgttatgaacatgttgccatgcatgtggaaaccaccctgtacatctatgtgcagagcttt
cagaatgcaaatcagaccgttagcatttgtggcagtctgccagaaccggcagagaaactgaccaaagcacctctgtgggtgaatg
tgccggaaaccgaaattgcagttggcatgccgctgaccacccagtataccagtgtgggtagcaatctgcagagcagtgatctgag
cgcacatgagaataccgatgaactgttctatttcgcatgggataatgagaaaccgatgcgtaagaaactggtgagcagctttagtat
tgccaatcgtccgattagtaatggtgaatatctggatttcatcaataagaaatccaagaccgaacgtgtttatccgaaacagtgggc
agaaattgatggtaccctgtatatccgtaccatgtatggcctgctgccgctggatgattatctgggctggccagttatgaccagttat
gatgatctgaacaattacgcaagtagccagggctgccgtctgccgaccgaagatgaactgaattgtttctatgatcgtgttctggaa
cgcaccgatgaaccgtatgtgagtaccgaaggcaaagccaccggctttcagcagctgcatccgctggcactgagcgataacag
cagtaatcagatctttaccggtgcctgggaatggaccagtaccgttctggagaaacatgaagatttcgaaccggaagaactgtatc
cggattacacccgtgatttctttgatggcaaacataatgtggtgctgggtggtagctttgccaccgcaacccgtattagtaatcgtcg
tagtttccgtaacttctaccaagccggttacaaatacgcctggattggtgcacgtctggtgaaaaactaa

The amino acid sequence of the *Schizosaccharomyces pombe* ergothioneine synthetase SPEGT2 is shown in SEQ ID NO: 3:

MAENNVYGHEMKKHFMLDPDYVNVNNGSCGTESLAVYNKHVQLLKEAQSK
PDFMCNAYMPMYMEATRNEVAKLIGADSSNIVFCNSATDGISTVLLTFPWEQNDEI
LMLNVAYPTCTYAADFAKNQHNLRLDVIDVGVEIDEDLFLKEVEQRFLQSKPRAFI
CDILSSMPVILFPWEKVVKLCKKYNIVSIIDGAHAIGHIPMNLANVDPDFLFTNAHK
WLNSPAACTVLYVSAKNHNLIEALPLSYGYGLREKESIAVDTLTNRFVNSFKQDLP
KFIAVGEAIKFRKSIGGEEKIQQYCHEIALKGAEIISKELGTSFIKPPYPVAMVNVEVP
LRNIPSIETQKVFWPKYNTFLRFMEFKGKFYTRLSGAVYLEESDFYYIAKVIKDF*;

The nucleotide sequence of its encoding gene (*spegt2*) is shown in SEQ ID NO: 4:

atggctgagaacaacgtgtacggccacgaaatgaaaaaacatttcatgctggatcctgactatgtaaacgtgaacaacgg
tagctgcggtaccgaatccctggctgtttacaacaaacacgttcagctgctgaaagaagctcagtccaaaccggacttcatgtgta
acgcttacatgccgatgtacatggaagcgacccgtaatgaagtcgccaaactgatcggtgcggactcttccaacatcgtgttctgc
aacagcgcaacggacggcatttctactgtcctgctgaccttcccgtgggagcagaacgatgaaatcctgatgctgaacgttgcgta
tccgacctgtacctacgctgcggactttgcgaaaaaccagcataacctgcgcctggacgttatcgatgttggtgttgaaatcgatga
agatctgtttctgaaagaagttgaacagcgcttcctgcagtccaaaccgcgtgcgttcatctgcgacatcctgtcctctatgccggtc
attctgtttccgtgggagaaagtggtgaagctgtgcaaaaaatacaatattgtgtccatcatcgacggtgcgcacgcgattggccac

atcccgatgaatctggctaacgtggatccggattttctgttcaccaacgcgcacaaatggctgaactctccggcagcgtgcaccgt
gctgtacgtttctgcaaagaaccacaacctgatcgaagcactgccactgagctacggctacggcctgcgtgaaaaagaatctattg
cagttgacaccctgaccaaccgcttcgttaacagcttcaaacaagatctgccgaaattcatcgcagtcggcgaagctatcaaattcc
gtaagagcatcggtggcgaagaaaaaatccagcagtactgtcacgaaatcgcgctgaaaggtgcggagattatctctaaagagc
tgggcacctccttcatcaaaccgccgtatccagttgccatggttaacgttgaggttccgctgcgtaacattccaagcatcgaaaccc
agaaagttttctggccgaaatataataccttcctgcgtttcatggaattcaaaggcaaattctacacccgtctgtctggcgccgtgtat
ctggaagaatctgacttctactatatcgccaaagtaatcaaggacttctgttccctgtaa;

The amino acid sequence of the *Schizosaccharomyces pombe* ergothioneine synthetase SPEGT1 after removing the catalytic methylation domain (SPEGT1-tr) is shown in SEQ ID NO: 5:

PAMLQLPLQPCPSLAEWDALRKVWLFITNKLLNKDNMYTAWIPLRHPPIFYIG
HVPVFNDIYLTKIVKNKATANKKHFWEWFQRGIDPDIEDPSKCHWHSEVPESWPSP
DQLREYEKESWEYHIVKLCKAMDELSTSEKRILWLCYEHVAMHVETTLYIYVQSFQ
NANQTVSICGSLPEPAEKLTKAPLWVNVPETEIAVGMPLTTQYTSVGSNLQSSDLSA
HENTDELFYFAWDNEKPMRKKLVSSFSIANRPISNGEYLDFINKKSKTERVYPKQW
AEIDGTLYIRTMYGLLPLDDYLGWPVMTSYDDLNNYASSQGCRLPTEDELNCFYDR
VLERTDEPYVSTEGKATGFQQLHPLALSDNSSNQIFTGAWEWTSTVLEKHEDFEPE
ELYPDYTRDFFDGKHNVVLGGSFATATRISNRRSFRNFYQAGYKYAWIGARLVKN*
;

The nucleotide sequence of its encoding gene (*spegt1 tr*) is shown in SEQ ID NO: 6*;*

ccagccatgctgcagctgccgctgcagccttgtccgagcctggcagaatgggatgccctgcgcaaagtgtggctgttcat
taccaacaaactgctgaataaggacaatatgtacaccgcctggattccgctgcgtcatccaccgatcttctacattggccatgtgcc
ggtgttcaacgatatctacctgaccaagattgtgaagaataaggcaaccgccaataagaaacatttctgggaatggtttcagcgtg
gtattgatccggatattgaagatccgagcaaatgccattggcatagtgaagttccggaaagttggccgtctccggatcagctgcgt
gaatatgagaaagaaagttgggaatatcatatcgtgaaactgtgtaaagcaatggatgaactgagtaccagtgagaaacgtattct
gtggctgtgttatgaacatgttgccatgcatgtggaaaccaccctgtacatctatgtgcagagctttcagaatgcaaatcagaccgtt
agcatttgtggcagtctgccagaaccggcagagaaactgaccaaagcacctctgtgggtgaatgtgccggaaaccgaaattgca
gttggcatgccgctgaccacccagtataccagtgtgggtagcaatctgcagagcagtgatctgagcgcacatgagaataccgatg
aactgttctatttcgcatgggataatgagaaaccgatgcgtaagaaactggtgagcagctttagtattgccaatcgtccgattagtaa
tggtgaatatctggatttcatcaataagaaatccaagaccgaacgtgtttatccgaaacagtgggcagaaattgatggtaccctgtat
atccgtaccatgtatggcctgctgccgctggatgattatctgggctggccagttatgaccagttatgatgatctgaacaattacgcaa
gtagccagggctgccgtctgccgaccgaagatgaactgaattgtttctatgatcgtgttctggaacgcaccgatgaaccgtatgtg
agtaccgaaggcaaagccaccggctttcagcagctgcatccgctggcactgagcgataacagcagtaatcagatctttaccggtg
cctgggaatggaccagtaccgttctggagaaacatgaagatttcgaaccggaagaactgtatccggattacacccgtgatttctttg
atggcaaacataatgtggtgctgggtggtagctttgccaccgcaacccgtattagtaatcgtcgtagtttccgtaacttctaccaagc
cggttacaaatacgcctggattggtgcacgtctggtgaaaaactaa;

The amino acid sequence of the mutant of *Schizosaccharomyces pombe* ergothioneine synthetase SPEGT1 (SPEGT1-tr M10) is shown in SEQ ID NO: 7:

PAMLQLPLQPCPSLAEWDALRKVWLFITNKLLNKDNMYTAWIPLRHPPILFIG
HVPVFNDIYLTKIVKNKATANKKHFWEWFQRGIDPDIEDPSKCNWNSEVPESWPSP
DQLREYEKESWEYHIVKLCKAMDELSTSEKRILWLCYEHVALHVETTLYIYVQSFQ
NANQTVSICGSLPEPAEKLTKAPLWVNVPETEIAVGMPLTTQYTSVGSNLQSSDLSA
HENTDELFYFAWDNEKPMRKKLVSSFSIANRPISNGEYLDFINKKSKTERVYPKQW
AEIDGTLYIRTMYGLLPLDDYLGWPVMTSYDDLNNYASSQGCRLPTEDELNCFYDR

VLERTDEPYVSTEGKATGFQQLHPLALSDNSSNQIFTGAWECTSTVLEKHEDFEPEE
LYPDYTRDFFDGKLNVVLGGSFATATRISNRRSLRNFYQAGYKSAWIGARLVKN*;

The nucleotide sequence of its encoding gene (*spegt1-tr M10*) is shown in SEQ ID NO: 8:

ccagccatgctgcagctgccgctgcagccttgtccgagcctggcagaatgggatgccctgcgcaaagtgtggctgttcat
taccaacaaactgctgaataaggacaatatgtacaccgcctggattccgctgcgtcatccaccgatcctcttcattggccatgtgcc
ggtattcaacgatatctacctgaccaagattgtgaagaataaggcaaccgccaataagaaacatttctgggaatggtttcagcgtgg
tattgatccggatattgaagatccgagcaaatgcaattggaacagtgaagttccggaaagttggccgtctccggatcagctgcgtg
aatatgagaaagaaagttgggaatatcatatcgtgaagctgtgtaaagcaatggatgaactgagtaccagtgagaaacgtattctgt
ggctgtgttatgaacatgttgccctgcatgtggaaaccaccctgtacatctatgtgcagagctttcagaatgcaaatcagaccgttag
catttgtggcagtctgccagaaccggcagagaaactgaccaaagcacctctgtgggtgaatgtgccggaaaccgaaattgcagtt
ggcatgccgctgaccacccagtataccagtgtgggtagcaatctgcagagcagtgatctgagcgcacatgagaataccgatgaa
ctgttctatttcgcatgggataatgagaaaccgatgcgtaagaaactggtgagcagctttagtattgccaatcgtccgattagtaatg
gtgaatatctggatttcatcaataagaaatccaagaccgaacgtgtttatccgaaacagtgggcagaaattgatggtaccctgtatat
ccgtaccatgtatggcctgctgccgctggatgattatctgggctggccagttatgaccagttatgatgatctgaacaattacgcaagt
agccagggctgccgtctgccgaccgaagatgaactgaattgtttctatgatcgtgttctggaacgcaccgatgaaccgtatgtgag
taccgaaggcaaagccaccggctttcagcagctgcatccgctggcactgagcgataacagcagtaatcagatctttaccggtgcc
tgggaatgtaccagtaccgttctggagaaacatgaagatttcgaaccggaagaactgtacccggattacacccgtgatttctttgat
ggcaaacttaatgtggtgctgggtggtagctttgccaccgcaacccgtattagtaatcgtcgtagtctccgtaacttctaccaagcc
ggttacaaatccgcctggattggtgcacgtctggtgaaaaactaa;

The amino acid sequence of the *Schizosaccharomyces pombe* ergothioneine synthetase SPEGT2 after directed evolution (SPEGT2 M3) is shown in SEQ ID NO: 9:'

MAENNVYGHEMKKHFMLDPDYVNVNNGPCGTESLAVYNKHVQLLKEAQSK
PDFMCNAYMPMYMEATRNEVAKLIGADSSNIVFCNSATDGISTVLLTFPWEQNDEI
LMLNVAFPTCTYAADFAKNQHNLRLDVIDVGVEIDEDLFLKEVEQRFLQSKPRAFI
CDILASMPVILFPWEKVVKLCKKYNIVSIIDGAHAIGHIPMNLANVDPDFLFTNAHK
WLNSPAACTVLYVSAKNHNLIEALPLSYGYGLREKESIAVDTLTNRFVNSFKQDLP
KFIAVGEAIKFRKSIGGEEKIQQYCHEIALKGAEIISKELGTSFIKPPYPVAMVNVEVP
LRNIPSIETQKVFWPKYNTFLRFMEFKGKFYTRLSGAVYLEESDFYYIAKVIKDFCSL
*;

The nucleotide sequence of its encoding gene (*spegt2 M3*) is shown in SEQ ID NO: 10:

Atggctgagaacaacgtgtacggccacgaaatgaaaaaacatttcatgctggatcctgactatgtaaacgtgaacaacg
gtccctgcggtaccgaatccctggctgtttacaacaaacacgttcagctgctgaaagaagctcagtccaaaccggacttcatgtgt
aacgcttacatgccgatgtacatggaagcgacccgtaatgaagtcgccaaactgatcggtgcggactcttccaacatcgtgttctg
caacagcgcaacggacggcatttctactgtcctgctgaccttcccgtgggagcagaacgatgaaatcctgatgctgaacgttgcgt
ttccgacctgtacctacgctgcggactttgcgaaaaaccagcataacctgcgcctggacgttatcgatgttggtgttgaaatcgatg
aagatctgtttctgaaagaagttgaacagcgcttcctgcagtccaaaccgcgtgcgttcatctgcgacatcctggcctctatgccgg
tcattctgtttccgtgggagaaagtggtgaagctgtgcaaaaaatacaatattgtgtccatcatcgacggtgcgcacgcgattggcc
acatcccgatgaatctggctaacgtggatccggattttctgttcaccaacgcgcacaaatggctgaactctccggcagcgtgcacc
gtgctgtacgtttctgcaaagaaccacaacctgatcgaagcactgccactgagctacggctacggcctgcgtgaaaaagaatctat
tgcagttgacaccctgaccaaccgcttcgttaacagcttcaaacaagatctgccgaaattcatcgcagtcggcgaagctatcaaatt
ccgtaagagcatcggtggcgaagaaaaaatccagcagtactgtcacgaaatcgcgctgaaaggtgcggagattatctctaaaga
gctgggcacctccttcatcaaaccgccgtatccagttgccatggttaacgttgaggttccgctgcgtaacattccaagcatcgaaac

ccagaaagttttctggccgaaatataataccttcctgcgtttcatggaattcaaaggcaaattctacacccgtctgtctggcgccgtgt
atctggaagaatctgacttctactatatcgccaaagtaatcaaggacttctgttccctgtaa;

In a preferred implementation, the amino acid sequences shown in SEQ ID NO: 7 and SEQ ID NO: 9 may have one or several amino acid residues substitutions, deletions, and/or additions, provided that their functions are not affected. Derivative proteins with similar functions are also within the scope of protection of the invention.

In another preferred embodiment, the nucleotide sequences shown in SEQ ID NO: 8 and SEQ ID NO: 10 may have one or several nucleotide substitutions, deletions, and/or additions, provided that their functions are not affected, and nucleotide sequences with similar functions are also within the scope of protection of the invention.

In the invention, there is also provided a combination of *Schizosaccharomyces pombe* ergothioneine synthetases, comprising the amino acid sequences of *Schizosaccharomyces pombe* ergothioneine synthetase SPEGT1 and SPEGT2 after removing demethylation domains and/or directed evolution. Specifically, the *Schizosaccharomyces pombe* ergothioneine synthetas SPEGT1 is SPEGT1-tr M10, whose amino acid sequence shown in SEQ ID NO: 7, and the *Schizosaccharomyces pombe* ergothioneine synthetase SPEGT2 is SPEGT2 M3, whose amino acid sequence shown in SEQ ID NO: 9. It should be understood that the above amino acid sequences may be substituted and/or deleted and/or added with one or several amino acid residues to form derivative proteins with essentially the same function, which can also be used as synthetic enzyme combinations in the invention.

In addition, recombinant vectors, expression kits, and recombinant bacteria containing said encoding gene are also within the scope of protection of the invention.

### A method for synthesizing ergothioneine

In the invention, there is also provided a method for synthesizing ergothioneine, which uses L-histidine as a raw material, and employs palladium-carbon catalyzed a reductive amination demethylation with formaldehyde, and undergoes a nucleophilic substitution with iodomethane to synthesize L-histidine betaine. Then, a combination of *Schizosaccharomyces pombe* ergothioneine synthetase described in the invention is used for enzyme catalytic preparation, so that ergothioneine can be obtained at high yield.

Specifically, the method comprises the steps of:

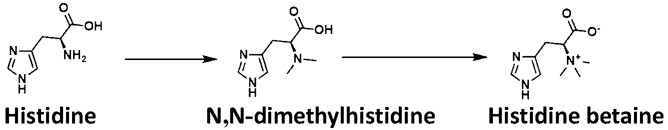

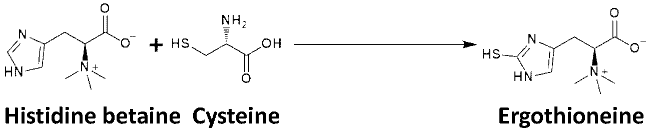

In the presence of the ergothioneine chemical-enzyme coupling system as described in the second aspect of the invention, ergothioneine is obtained from L-histidine and cysteine.

Preferably, the enzyme catalytic step is carried out in the presence of Fe²⁺, pyridoxal phosphate, and thiol or phosphine reducing agents.

In order to obtain the best reaction results, the method is carried out in a buffer system with pH=7-9 at 20-30 °C.

**The invention has the following advantages and effects compared to the prior art:**
The invention uses chemical methods to synthesize L-histidine betaine on a large scale, overcoming the problem of low catalytic methylation efficiency of ergothioneine synthetase EGT1 in E. coli fermentation broth (mainly due to the low concentration of S-adenosylmethionine in E. coli). Then, starting from the ergothioneine synthetase of *Schizosaccharomyces pombe,* the *Schizosaccharomyces pombe* ergothioneine synthetase SPEGT1 (Uniprot ID:O94632) and SPEGT2 (Uniprot ID: 094431) was obtained on Uniprot, and the information from Uniprot was used to remove the SPEGT1 catalytic methylation domain (M1~F328) to obtain the amino acid sequence of SPEGT1 tr. The above-mentioned genes were synthesized and constructed into the pET28A vector, and heterologous expression was achieved in E. coli. enzymes (SPEGT1-tr M10 and SPEGT2 M3) required for the synthesis of ergothioneine with significantly increased activity was obtain by directed evolution of coding genes (spegt1-tr, spegt2) related to *Schizosaccharomyces pombe* ergothioneine synthesis using random mutation kit, which were used for the synthesis of ergothioneine. Compared with the existing synthetic ergothioneine technology, the yield is significantly improved. At the same time, the genetically engineered E. coli constructed by the invention is safe and stable, with a short production cycle, demonstrating the enormous potential in fermentation amplification of SPEGT1-tr M10 and SPEGT2 M3 engineering enzymes and industrial production of ergothioneine.

The invention will be further explained in conjunction with specific examples. It should be understood that these examples are only used to illustrate the invention and not to limit the scope of the invention. The experimental methods without specific conditions specified in the following examples are usually carried out under conventional conditions or conditions recommended by the manufacturer. Unless otherwise specified, percentages and portions are calculated by weight. Other materials and reagents used, unless otherwise specified, are obtained from commercial sources.

### Example 1

### The synthesis of N,N-dimethyl histidine

To a 1L high-pressure reactor was added 625ml of water, 96g (0.5mol) of L-histidine hydrochloride, 96g (1.185mol, 2.37eq) of 37% formaldehyde aqueous solution, and 10g of 10% Pd-C catalyst in sequence. After addition, replaced with nitrogen three times, and then replaced with hydrogen three times. After the replacement is completed, hydrogenation was start with a hydrogen gas flow at a maintaind pressure of 0.5-1.0MPa, the reaction temperature was controlled at 10 °C~30 °C, and reacted for 24 hours. After the reaction was complete, the catalyst was filtered off, and the filtrate was concentrated under reduced pressure to about (100-150) ml. 600L of ethanol was added and recrystallized to obtain about 85g of the product, with a yield of 71%. ¹H NMR (D₂O) δ 8.51 (s, 1H), 7.26 (s, 1H), 3.76-3.80 (m, 1H), 3.31-3.36 (m, 1H), 3.15-3.21 (s, 1H), 2.83 (s, 6H).

### Example 2

### Synthesis of Histidine Betaine

238g (1mol) of N, N-dimethyl histidine was suspended in 1500ml methanol and a solution of 150g of concentrated ammonia water/35ml of water was added dropwise at a controlled temperature below 15 °C. After addtion, pH=9, 195g (1.37mol, 1.37eq) of iodomethane was added dropwise. After the dropwise addition was complete, the reaction was carried out at 15 °C~25 °C for 4-5 hours. Then, methanol was evaporated under reduced pressure and diluted with water to about 4000ml, the pH was adjusted to 9-11 using a solution of 40g sodium hydroxide/100ml water. The solution was then subjected to membrane separation and anion exchange resin desalination, and the desalinated aqueous solution was concentrated under reduced pressure to about 250ml, then 800ml of isopropanol was added and stirred overnight. The solid was precipitated, filtered, and dried to obtain 150g of histidine betaine with a yield of 76%. ¹H NMR(D₂O) δ7.73(s, 1H), 7.04(s, 1H), 3.92-3.96(m, 1H), 3.20-3.26(m, 11H).

### Example 4

### Construction of ergothioneine synthesis plasmid

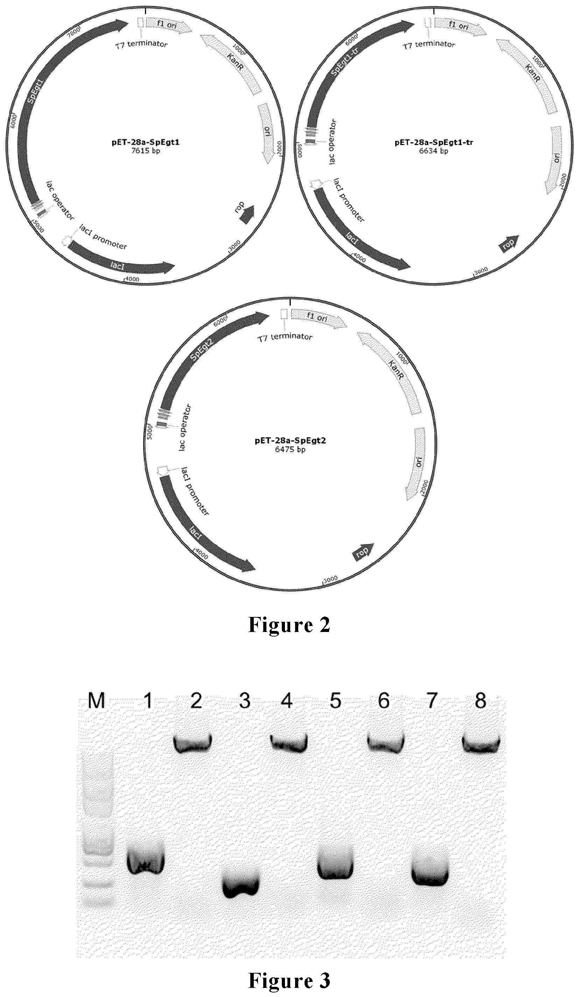

Uniprot was used to search EGT1 and EGT2, locate the *Schizosaccharomyces pombe* ergothioneine synthetases SPEGT1 (Uniprot ID: O94632) and SPEGT2 (Uniprot ID: 094431), as shown in SEQ ID No: 1 and 3. Due to the enzyme catalytic step of the invention uses histidine betaine as a raw material, the catalytic methylation domain on SPEGT1 does not need to be used and can be removed. According to the AlphaFold structure on Uniprot, cleavage was performed between the two catalytic domains to obtain the histidine betaine cysteine sulfoxide synthase SPEGT1-tr from P329 to N773, as shown in SEQ ID No.: 5. Nucleotide sequences of the above three protein sequences can be expressed in general biooptimized E. coli (such as SEQ ID No. 2, 4, 6), and inserted into the vector pET28A between NdeI and XhoI to synthesize plasmids pET-28a-SpEgt1, pET-28a-SpEgt1-tr, and pET-28a-SpEgt2(as shown in Figure 2).

### Example 5

### Optimization of the combination of ergothioneine synthases EGT1 and EGT2

Using SPEGT1-tr and SPEGT2 as templates, ergothioneine synthases EGT1-tr and EGT2 from 9 other strains were obtained from NCBI protein blast.

The reaction system is 10mM histidine betaine, 15mM cysteine, 100mM Tris (pH 8.0), 10mM Fe²⁺, 1mM pyridoxal phosphate (PLP), 10mM beta mercaptoethanol, 10% (v/v) EGT1-tr lysate, and 10% (v/v) EGT2 lysate. Among them, there are 100 combinations of ergothionein synthase EGT1 tr and EGT2, including orthogonal combinations of 10 EGT1 and 10 EGT2, the specific sequences are shown below. The reaction procedure is reacted on a 500rpm shaker at 25 °C for 2 hours. After 0.1mL of the reaction is complete, 10mM (final concentration) internal standard para aminobenzoic acid and 0.3mL solution of 0.1% (v/v) trifluoroacetic acid in acetonitrile were added, centrifuged and the supernatant was collected for HPLC analysis. HPLC conditions are C18 analytical column; phase A: 50mM ammonium acetate, phase B: 100% acetonitrile; chromatographic conditions: 0%~70% phase B for 3.5 minutes, 70%~0% phase B for 1 minute, 0% phase B for 0.5 minutes; the detection wavelength is 254nm and the flow rate is 1mL/min. The reaction yield of the optimal combination of SPEGT1-tr (SEQ ID NO: 5) and SPEGT2 (SEQ ID NO: 3) is 7%, while the reaction yield of the combination of full-length SPEGT1 and SPEGT2 is 0%. This demonstrates the importance of EGT1 truncation in removing methylation catalytic domains for catalytic reactions in cracking solutions.

The amino acid sequences and nucleotide sequences of 10 EGT1-tr encoding genes are shown in SEQ ID No. 5-6 and 11-28, and the amino acid sequences and nucleotide sequences of 10 EGT2 encoding genes are shown in SEQ ID No. 3-4 and 29-46. The screening results are shown in Table 1, where SPEGT1-tr and SPEGT2 are the best combinations.

The amino acid sequences of EGT1-tr from 9 other strains and the nucleotide sequences of their encoding genes.
SEQ ID NO: 11
   Type: Protein XP_013019833.1 P331~I767
   Name: SOcEGT1-tr
   Source: *Schizosaccharomyces octosporus* yFS286 
PILPQIPKEASISLEEWSQTRDIWLFVTNKLLNDSNIFNVWIPLRHPFIFYMGHIP
VFNDIYLSRIFENPATASKREYWDWFQRGIDPDVENPEQCHWHSQTPPKWPSPNEL
RSYEVASWQNHILKLLDGSHALSPSQKRILWLCYEHVAMHIETTLYIYVQSFQHPK
QNNTLCGLPPSKNLKLKKDPSWIKFPNAQVLQGLPIRSDQKTKLNSEEPDEQEFFG
WDNEKPLRMKQIPSFQIANRPISNGEYLDYLESKPADDKHYPKSWKVIDGKLYVTT
MYGLLPLESYHSWPVMASFEELNDYAASKGCRLPTEEELNHFYDHVLHRKSETYV
STKGMATGFQQLHPANLKDDGTHQIFTGAWEWSSTVLDKHEGFEPEALYPDYTKD
FFDGKHNVVLGGSFATVPRIANRRSFRNFYQRQYQYAWITARLAKSI
SEQ ID NO: 12
   Type: DNA
   Name: *SOcEGT1-tr*
   Source: *Schizosaccharomyces octosporus* yFS286 
ccgattctgccgcagattccgaaagaagcgagcattagcctggaagaatggagccagacccgcgatatttggctgtttgt
gaccaacaaactgctgaacgatagcaacatttttaacgtgtggattccgctgcgccatccgtttattttttatatgggccatattccggt
gtttaacgatatttatctgagccgcatttttgaaaacccggcgaccgcgagcaaacgcgaatattgggattggtttcagcgcggcat
tgatccggatgtggaaaacccggaacagtgccattggcatagccagaccccgccgaaatggccgagcccgaacgaactgcgc
agctatgaagtggcgagctggcagaaccatattctgaaactgctggatggcagccatgcgctgagcccgagccagaaacgcatt
ctgtggctgtgctatgaacatgtggcgatgcatattgaaaccaccctgtatatttatgtgcagagctttcagcatccgaaacagaaca
 
acaccctgtgcggcctgccgccgagcaaaaacctgaaactgaaaaaagatccgagctggattaaatttccgaacgcgcaggtgc
tgcagggcctgccgattcgcagcgatcagaaaaccaaactgaacagcgaagaaccggatgaacaggaattttttggctgggata
acgaaaaaccgctgcgcatgaaacagattccgagctttcagattgcgaaccgcccgattagcaacggcgaatatctggattatctg
gaaagcaaaccggcggatgataaacattatccgaaaagctggaaagtgattgatggcaaactgtatgtgaccaccatgtatggcc
tgctgccgctggaaagctatcatagctggccggtgatggcgagctttgaagaactgaacgattatgcggcgagcaaaggctgcc
gcctgccgaccgaagaagaactgaaccatttttatgatcatgtgctgcatcgcaaaagcgaaacctatgtgagcaccaaaggcat
ggcgaccggctttcagcagctgcatccggcgaacctgaaagatgatggcacccatcagatttttaccggcgcgtgggaatggag
cagcaccgtgctggataaacatgaaggctttgaaccggaagcgctgtatccggattataccaaagatttttttgatggcaaacataa
cgtggtgctgggcggcagctttgcgaccgtgccgcgcattgcgaaccgccgcagctttcgcaacttttatcagcgccagtatcagt
atgcgtggattaccgcgcgcctggcgaaaagcatt
SEQ ID NO: 13
   Type: Protein XP_013021279.1 P330~I768
   Name: SCrEGT1-tr
   Source: *Schizosaccharomyces cryophilus* OY26 
PKLPQLPKEAPISLREWSGIRDIWIFITNKLLNDSNIFKVWIPLRHPFVFYMGHIP
VFNDIYLSRIFENPVTASKRQYWDWFQRGIDPDVENPENCHWHSQIPTNWPSPNEL
RSYEIASWQNHILKLYDGSHTLSPFQKRIVWLCYEHVAMHIETTLYIYVQSFQDPKQ
NNFICGLPPCKIPKLKKDPSWIKYYNGQVLLGLPVRNDRYSNSNPEEPDEQKFFGW
DNEKPSRTNHVPSFQIANRPISNGEYLNFVNNKPEGDRRYPKLWKLIDGKLYLKTM
YGLLPLDNYLSWPVMASFDELNAYAAFKGCRLPTEEELNYYYDQVLHRKSEAYVS
TEGMATGFQQLHPAKLKDDETPQIFTGAWEWTSTVLEKHEGFEPEALYPDYTKDFF
DGKHNVVLGGSFATVPRIANRRSFRNFYQRQYEYAWITARLAKGI
SEQ ID NO: 14
   Type: DNA
   Name: *SCrEGT1-tr*
   Source: *Schizosaccharomyces cryophilus* OY26 
ccgaaactgccacaactgccgaaagaagcaccaatcagcctgcgcgagtggagcggcatccgtgatatctggatcttca
tcaccaacaaactgctgaacgatagcaacatcttcaaagtctggatccctctgcgccatccgttcgttttctatatgggtcacatccc
ggtatttaacgacatctacctgtctcgcatcttcgagaacccggtgactgcgtctaaacgtcagtactgggattggtttcagcgtggt
attgatccggatgttgaaaatccggaaaactgccactggcactctcagatcccgactaactggccgagcccgaacgaactgcgtt
cctatgaaatcgcatcttggcagaaccacatcctgaaactgtatgatggtagccacaccctgtccccatttcagaagcgtatcgtat
ggctgtgctacgagcacgtggcaatgcacatcgaaactacgctgtacatttacgtgcagagcttccaggatccaaagcagaacaa
cttcatctgtggcctgccgccgtgcaaaatcccaaaactgaaaaaagacccgagctggattaaatactacaacggtcaggttctgc
tgggcctgccggtacgtaatgaccgttactctaactccaaccctgaagaaccggacgaacagaagttcttcggctgggataacga
aaagccgtctcgtaccaaccacgttccgtcttttcagattgcgaaccgtccgatttctaacggtgagtatctgaactttgtgaacaac
aaaccggaaggcgatcgtcgttacccgaaactgtggaaactgatcgacggtaaactgtacctgaaaaccatgtacggcctgctgc
cgctggacaactacctgagctggccagttatggcttctttcgacgaactgaatgcgtacgcagcgtttaaaggctgtcgtctgccaa
ccgaagaagaactgaactactactacgaccaggttctgcatcgtaaatccgaagcatacgtttccaccgaaggtatggccactggt
ttccagcagctgcatccggccaaactgaaagatgatgaaactccgcagatttttacgggtgcttgggaatggaccagcactgttct
ggagaaacacgaaggtttcgaacctgaagcgctgtatccagactataccaaagactttttcgacggcaaacacaacgtggttctg
ggcggttctttcgctaccgttcctcgcattgctaatcgtcgcagcttccgtaatttctatcagcgccagtacgaatatgcgtggattac
ggcccgtctggccaagggtatc
SEQ ID NO: 15
   Type: Protein WBW72835.1 P331~I767
   Name: SOsEGT1-tr
   Source: *Schizosaccharomyces osmophilus* 
PNLPQVPNEAPISLEEWSQTRAIWLFVTNKLLNDSNIFNVWIPLRHPFVFYMGH
IPVFNDIYLSRIFENPATAPKREYWDWFQRGIDPDVENPEHCHWHSQAPPNWPSPNE
LRSYEVASWQNHILKLFDGSHALSPSQKRIVWLCYEHVAMHIETTLYIYVQSFQHP
KQNNVICGLPPCKTPKLEKDPSWIKFSNGQVLLGLPIQNDRNTSSNPEKPDEQEFFG
WDNEKPSRIEQVSPFQIANRPISNGEYLDYLESKPASDKHYPKLWKLIDGKLYLKTM
YGLLPLENYSSWPVMASFEELNAYAASKGCRLPTEEELNYFYDHVLQRKSEAYVST
RGLATGFQQLHPAKLKDDGTPQIFTGAWEWSSTVLDKHEGFEPEALYPDYTQDFFD
GKHNVVLGGSFATVPRIANRRSFRNFYQRQYQYAWITARLAKSI
SEQ ID NO: 16
   Type: DNA
   Name: *SOsEGT1-tr*
   Source: *Schizosaccharomyces osmophilus* 
ccgaacctgccgcaggtgccgaacgaagcgccgattagcctggaagaatggagccagacccgcgcgatttggctgttt
gtgaccaacaaactgctgaacgatagcaacatttttaacgtgtggattccgctgcgccatccgtttgtgttttatatgggccatattcc
ggtgtttaacgatatttatctgagccgcatttttgaaaacccggcgaccgcgccgaaacgcgaatattgggattggtttcagcgcgg
cattgatccggatgtggaaaacccggaacattgccattggcatagccaggcgccgccgaactggccgagcccgaacgaactgc
gcagctatgaagtggcgagctggcagaaccatattctgaaactgtttgatggcagccatgcgctgagcccgagccagaaacgca
ttgtgtggctgtgctatgaacatgtggcgatgcatattgaaaccaccctgtatatttatgtgcagagctttcagcatccgaaacagaa
caacgtgatttgcggcctgccgccgtgcaaaaccccgaaactggaaaaagatccgagctggattaaatttagcaacggccaggt
gctgctgggcctgccgattcagaacgatcgcaacaccagcagcaacccggaaaaaccggatgaacaggaattttttggctggga
taacgaaaaaccgagccgcattgaacaggtgagcccgtttcagattgcgaaccgcccgattagcaacggcgaatatctggattat
ctggaaagcaaaccggcgagcgataaacattatccgaaactgtggaaactgattgatggcaaactgtatctgaaaaccatgtatg
gcctgctgccgctggaaaactatagcagctggccggtgatggcgagctttgaagaactgaacgcgtatgcggcgagcaaaggc
tgccgcctgccgaccgaagaagaactgaactatttttatgatcatgtgctgcagcgcaaaagcgaagcgtatgtgagcacccgcg
gcctggcgaccggctttcagcagctgcatccggcgaaactgaaagatgatggcaccccgcagatttttaccggcgcgtgggaat
ggagcagcaccgtgctggataaacatgaaggctttgaaccggaagcgctgtatccggattatacccaggatttttttgatggcaaa
cataacgtggtgctgggcggcagctttgcgaccgtgccgcgcattgcgaaccgccgcagctttcgcaacttttatcagcgccagt
atcagtatgcgtggattaccgcgcgcctggcgaaaagcatt
SEQ ID NO: 17
   Type: Protein XP_002172097.1 F328~S758
   Name: SJEGT1-tr
   Source: *Schizosaccharomyces japonicus* yFS275 
FDVSRNPETPCPTLEEWTQIRLAWLYLVFKLYPRDLYFTELIPVRHPFIFYIGHV
PAFNDIYLARLTDGKPTLGRKDYWDWFQRGIDPDLDNTKKCHWHSQPPEKWPSVE
EVNEYERNVWSRLVSIYKQGEMSANMQRAMWMIYEHTAMHLETSYYILLQSDYHI
ISPNNFPPPIAPALQTDPTWVRVPESFITMGIPTTADGKETFYYGWDNEKPERQVSVR
CFEIANRPISNGEYLSFLKETTQSKEEFEAAIPKTWLLKDEMLFAKSMYGPLPIEHVL
 
GWPVATSYDELKQYANAKGCRLPTDYELRAFYDHVLKPNEETYVDTAGYATAFQ
QWYPKSLQDEEKPQIYTGLWEWTSTVLKEDLDFTPEELYPDYTRDFFDGKHNVVM
GGSFTTVARIANRRSFRNFYQRKYPYAWIGARLVKVTNTLS
SEQ ID NO: 18
   Type: DNA
   Name: SJEGT1-tr
   Source: *Schizosaccharomyces japonicus* yFS275 
tttgatgtgagccgtaacccggaaaccccgtgcccgaccctggaagaatggacccagattcgcctggcgtggctgtatct
ggtgtttaaactgtatccgcgcgatctgtattttaccgaactgattccggtgcgccatccgtttattttttatattggccatgtgccggcg
tttaacgatatttatctggcgcgcctgaccgatggcaaaccgaccctgggccgcaaagattattgggattggtttcagcgcggcatt
gatccggatctggataacaccaaaaaatgccattggcatagccagccgccggaaaaatggccgagcgtggaagaagtgaacga
atatgaacgcaacgtgtggagccgcctggtgagcatttataaacagggcgaaatgagcgcgaacatgcagcgcgcgatgtggat
gatttatgaacataccgcgatgcatctggaaaccagctattatattctgctgcagagcgattatcatattattagcccgaacaactttc
cgccgccgattgcgccggcgctgcagaccgatccgacctgggtgcgcgtgccggaaagctttattaccatgggcattccgacca
ccgcggatggcaaagaaaccttttattatggctgggataacgaaaaaccggaacgccaggtgagcgtgcgctgctttgaaattgc
gaaccgcccgattagcaacggcgaatatctgagctttctgaaagaaaccacccagagcaaagaagaatttgaagcggcgattcc
gaaaacctggctgctgaaagatgaaatgctgtttgcgaaaagcatgtatggcccgctgccgattgaacatgtgctgggctggccg
gtggcgaccagctatgatgaactgaaacagtatgcgaacgcgaaaggctgccgcctgccgaccgattatgaactgcgcgcgtttt
atgatcatgtgctgaaaccgaacgaagaaacctatgtggataccgcgggctatgcgaccgcgtttcagcagtggtatccgaaaag
cctgcaggatgaagaaaaaccgcagatttataccggcctgtgggaatggaccagcaccgtgctgaaagaagatctggattttacc
ccggaagaactgtatccggattatacccgcgatttttttgatggcaaacataacgtggtgatgggcggcagctttaccaccgtggc
gcgcattgcgaaccgccgcagctttcgcaacttttatcagcgcaaatatccgtatgcgtggattggcgcgcgcctggtgaaagtga
ccaacaccctgagc
SEQ ID NO: 19
   Type: Protein CCG81205.1 F324~V749
   Name: TDEGT1-tr
   Source: *Taphrina deformans* PYCC 5710 
FSSNPRDIGPVPTLEEWSELWKLWDVITMEMVPREMLESKPIDLRNPCIFYVG
HIPTFLDIHLSRVGNGRYLNPAYTQIFERGIDPDVDDPSQCHDHSSLPDKWPDLSEM
LSFRDQVRKRLRDVYASGSINDRKVARAVFTVYEHEAMHIETFLYMHLQADWTLS
PPKMLPPRFEEKPKEVGPASWMKMSPTTMNVGMNDVEGSDEGDYFGWDNEKPRR
STGLQPFTIQSRPVTNGEYAKYLNQTTEEGKRWRHPKSWTPDMRVKTPFGPIPLAA
AVNWPVAASYDELEKYANWCGGRLPTHDELRHFIDSSCDTDDARGTPFNDVHGK
KVNFSQWFPGNVEDSSKPQVYCGVWEWTSTPFARTPGFVTSQIYPGYSEDFFDGKH
NIVLGGSWATISRIAARKSFVNWYQRNYEYAWTGCRLVKDV
SEQ ID NO: 20
   Type: DNA
   Name: *TDEGT1-tr*
   Source: *Taphrina deformans* PYCC 5710 
tttagcagcaacccgcgcgatattggcccggtgccgaccctggaagaatggagcgaactgtggaaactgtgggatgtga
ttaccatggaaatggtgccgcgcgaaatgctggaaagcaaaccgattgatctgcgcaacccgtgcattttttatgtgggccatattc
 
cgacctttctggatattcatctgagccgcgtgggcaacggccgctatctgaacccggcgtatacccagatttttgaacgcggcattg
atccggatgtggatgatccgagccagtgccatgatcatagcagcctgccggataaatggccggatctgagcgaaatgctgagctt
tcgcgatcaggtgcgcaaacgcctgcgcgatgtgtatgcgagcggcagcattaacgatcgcaaagtggcgcgcgcggtgtttac
cgtgtatgaacatgaagcgatgcatattgaaacctttctgtatatgcatctgcaggcggattggaccctgagcccgccgaaaatgct
gccgccgcgctttgaagaaaaaccgaaagaagtgggcccggcgagctggatgaaaatgagcccgaccaccatgaacgtgggc
atgaacgatgtggaaggcagcgatgaaggcgattattttggctgggataacgaaaaaccgcgccgcagcaccggcctgcagcc
gtttaccattcagagccgcccggtgaccaacggcgaatatgcgaaatatctgaaccagaccaccgaagaaggcaaacgctggc
gccatccgaaaagctggaccccggatatgcgcgtgaaaaccccgtttggcccgattccgctggcggcggcggtgaactggccg
gtggcggcgagctatgatgaactggaaaaatatgcgaactggtgcggcggccgcctgccgacccatgatgaactgcgccatttt
attgatagcagctgcgataccgatgatgcgcgcggcaccccgtttaacgatgtgcatggcaaaaaagtgaactttagccagtggtt
tccgggcaacgtggaagatagcagcaaaccgcaggtgtattgcggcgtgtgggaatggaccagcaccccgtttgcgcgcaccc
cgggctttgtgaccagccagatttatccgggctatagcgaagatttttttgatggcaaacataacattgtgctgggcggcagctggg
cgaccattagccgcattgcggcgcgcaaaagctttgtgaactggtatcagcgcaactatgaatatgcgtggaccggctgccgcct
ggtgaaagatgtg
SEQ ID NO: 21
   Type: Protein XP_040724913.1 L324~L745
   Name: PLEGT1-tr
   Source: *Protomyces lactucae-debilis* 
LSRQADTLKPVPSLAEWEQLWKLWDTITLGMIPQEMLLCKPIDLRHPCIFYLG
HIPTFLDIHLTRVGNGKYTNDAYTRIFERGIDPDVDDPTHCAHSDVPDTWPDLQEI
LAFRDTVRNRLREVYRQDTASFNRQLARALFVTFEHEAMHAETLLYMLLQSAETR
PPPGVLSSLPTAAAPELEPAAWITLETSNFEVGMDDPEEAESGQYFGWDNEKPRRSI
SHAKVAIQARPISNGEYAAFLDAHPKLAAPASWTQSREVKTVFGPVPLTYAADWPV
AASYDELQAYAKWKDARLPSFAELRHFLDDHSGESSNEPFNNVLDKNVNFGMWH
PSSLTSSRESVQTYTGFWEWTSTEFSAPKGFVPSKVYPAYSSDFFDGKHNIVLGGSW
ATITRIAARKSFVNWYQRNYKFAWTSGRLARDL
SEQ ID NO: 22
   Type: DNA
   Name: *PLEGT1-tr*
   Source: *Protomyces lactucae-debilis* 
ctgagccgccaggcggataccctgaaaccggtgccgagcctggcggaatgggaacagctgtggaaactgtgggatac
cattaccctgggcatgattccgcaggaaatgctgctgtgcaaaccgattgatctgcgccatccgtgcattttttatctgggccatattc
cgacctttctggatattcatctgacccgcgtgggcaacggcaaatataccaacgatgcgtatacccgcatttttgaacgcggcattg
atccggatgtggatgatccgacccattgccatgcgcatagcgatgtgccggatacctggccggatctgcaggaaattctggcgttt
cgcgataccgtgcgcaaccgcctgcgcgaagtgtatcgccaggataccgcgagctttaaccgccagctggcgcgcgcgctgttt
gtgacctttgaacatgaagcgatgcatgcggaaaccctgctgtatatgctgctgcagagcgcggaaacccgcccgccgccggg
cgtgctgagcagcctgccgaccgcggcggcgccggaactggaaccggcggcgtggattaccctggaaaccagcaactttgaa
gtgggcatggatgatccggaagaagcggaaagcggccagtattttggctgggataacgaaaaaccgcgccgcagcattagcca
tgcgaaagtggcgattcaggcgcgcccgattagcaacggcgaatatgcggcgtttctggatgcgcatccgaaactggcggcgc
cggcgagctggacccagagccgcgaagtgaaaaccgtgtttggcccggtgccgctgacctatgcggcggattggccggtggc
ggcgagctatgatgaactgcaggcgtatgcgaaatggaaagatgcgcgcctgccgagctttgcggaactgcgccattttctggat
gatcatagcggcgaaagcagcaacgaaccgtttaacaacgtgctggataaaaacgtgaactttggcatgtggcatccgagcagc
 
ctgaccagcagccgcgaaagcgtgcagacctataccggcttttgggaatggaccagcaccgaatttagcgcgccgaaaggcttt
gtgccgagcaaagtgtatccggcgtatagcagcgatttttttgatggcaaacataacattgtgctgggcggcagctgggcgaccat
tacccgcattgcggcgcgcaaaagctttgtgaactggtatcagcgcaactataaatttgcgtggaccagcggccgcctggcgcgc
gatctg
SEQ ID NO: 23
   Type: Protein XP_019024945.1 H331~N798
   Name: SCoEGT1-tr
   Source: *Saitoella complicata* NRRL Y-17804 
HIPLLGSKEPVPTTQDWSDLWSAWDTVTLQMIPQKMLLTKPIDLRHPCIFYIG
HIPTFLDIHLTRAKLGGDAYINAAYTRIFERGIDPDVDDPTQCHVHSEVPDSWPDLQ
ELLEFRDEVRARVKNCYGANGLVSSALDHVRRRALWLAFEHEAMHLETLLYMLL
QSPQTVPPPGVPVPDWKLLASASQRPTATKEEDKWMMVESRKTNVGLNDPEGAEN
GGTDGRLMGWDNEKPQREWTVNGFIASKRPITNGEYATFLQESGSTKIPESWSRVD
GVYKVKTVFGLVAMEHAHDWPVIGSYDEIASYATYAGGRLPREEELRALYDVIAD
SSATPGTLIAGVNGHLTSNGVHTTPPAVPNFIDLSGANVGFKHWHPTPAHATQLVG
SGGVGAWEWTSTPMAPHEGFVPSKVYSGYTADFFDGKHNVVLGGSWATHGRLAG
RRTFRNWYQRNYGFAWVTGRVIKDIDMN
SEQ ID NO: 24
   Type: DNA
   Name: *SCoEGT1-tr*
   Source: *Saitoella complicata* NRRL Y-17804 
catattccgctgctgggcagcaaagaaccggtgccgaccacccaggattggagcgatctgtggagcgcgtgggatacc
gtgaccctgcagatgattccgcagaaaatgctgctgaccaaaccgattgatctgcgccatccgtgcattttttatattggccatattcc
gacctttctggatattcatctgacccgcgcgaaactgggcggcgatgcgtatattaacgcggcgtatacccgcatttttgaacgcgg
cattgatccggatgtggatgatccgacccagtgccatgtgcatagcgaagtgccggatagctggccggatctgcaggaactgctg
gaatttcgcgatgaagtgcgcgcgcgcgtgaaaaactgctatggcgcgaacggcctggtgagcagcgcgctggatcatgtgcg
ccgccgcgcgctgtggctggcgtttgaacatgaagcgatgcatctggaaaccctgctgtatatgctgctgcagagcccgcagacc
gtgccgccgccgggcgtgccggtgccggattggaaactgctggcgagcgcgagccagcgcccgaccgcgaccaaagaaga
agataaatggatgatggtggaaagccgcaaaaccaacgtgggcctgaacgatccggaaggcgcggaaaacggcggcaccga
tggccgcctgatgggctgggataacgaaaaaccgcagcgcgaatggaccgtgaacggctttattgcgagcaaacgcccgatta
ccaacggcgaatatgcgacctttctgcaggaaagcggcagcaccaaaattccggaaagctggagccgcgtggatggcgtgtat
aaagtgaaaaccgtgtttggcctggtggcgatggaacatgcgcatgattggccggtgattggcagctatgatgaaattgcgagcta
tgcgacctatgcgggcggccgcctgccgcgcgaagaagaactgcgcgcgctgtatgatgtgattgcggatagcagcgcgaccc
cgggcaccctgattgcgggcgtgaacggccatctgaccagcaacggcgtgcataccaccccgccggcggtgccgaactttatt
gatctgagcggcgcgaacgtgggctttaaacattggcatccgaccccggcgcatgcgacccagctggtgggcagcggcggcgt
gggcgcgtgggaatggaccagcaccccgatggcgccgcatgaaggctttgtgccgagcaaagtgtatagcggctataccgcgg
atttttttgatggcaaacataacgtggtgctgggcggcagctgggcgacccatggccgcctggcgggccgccgcacctttcgcaa
ctggtatcagcgcaactatggctttgcgtgggtgaccggccgcgtgattaaagatattgatatgaac
SEQ ID NO: 25
   Type: Protein ODV92841.1 H330~K792
   Name: TCEGT1-tr
   Source: *Tortispora caseinolytica* NRRL Y-17796 
HLVYKPECRLPLASVVANMPSLEEWAYMHNMWDYVTCKMAPESMYLEQPI
YLRNTVIFYLGHSPCFLDVVLNRANTGTLATQPVWYQDIFARGIDPVDDPSKVHS
HSSIPAEWPPLDDILQYQDSVRSRLRRLLTDNTGKMSSRTARAIWLGYEHDAMHLE
TLMYMMLQSPLKLSPPGVPKPSFIQSKHANSNQKWIRIPATVINIGIDDPDEADHRPG
SHASRLMGWDNEKPSRGSVTVPPFSASPSLITNRQYYSYLLDRLRSGVVIKPPESWN
ASEDLQNVYVKTFFGDAPLCVDGSLNSDFIASDWPVCASYNELQEFMAQFQNGEF
RFPTEHEVLAIYKTQQQRLHNDDPDYYDLSDSNVGFKHWHPSSNVDCIEGISGPGG
QGVWEWTSTVFDSHEGFTVNELYPEYSADFFDGKHNVVLGGSWATAPRLSGRRSF
RNWYQRSYCFAWIGARMVRK
SEQ ID NO: 26
   Type: DNA
   Name: *TCEGT1-tr*
   Source: *Tortispora caseinolytica* NRRL Y-17796 
catctggtgtataaaccggaatgccgcctgccgctggcgagcgtggtggcgaacatgccgagcctggaagaatgggcg
tatatgcataacatgtgggattatgtgacctgcaaaatggcgccggaaagcatgtatctggaacagccgatttatctgcgcaacacc
gtgattttttatctgggccatagcccgtgctttctggatgtggtgctgaaccgcgcgaacaccggcaccctggcgacccagccggt
gtggtatcaggatatttttgcgcgcggcattgatccggatgtggatgatccgagcaaagtgcatagccatagcagcattccggcgg
aatggccgccgctggatgatattctgcagtatcaggatagcgtgcgcagccgcctgcgccgcctgctgaccgataacaccggca
aaatgagcagccgcaccgcgcgcgcgatttggctgggctatgaacatgatgcgatgcatctggaaaccctgatgtatatgatgct
gcagagcccgctgaaactgagcccgccgggcgtgccgaaaccgagctttattcagagcaaacatgcgaacagcaaccagaaat
ggattcgcattccggcgaccgtgattaacattggcattgatgatccggatgaagcggatcatcgcccgggcagccatgcgagcc
gcctgatgggctgggataacgaaaaaccgagccgcggcagcgtgaccgtgccgccgtttagcgcgagcccgagcctgattacc
aaccgccagtattatagctatctgctggatcgcctgcgcagcggcgtggtgattaaaccgccggaaagctggaacgcgagcgaa
gatctgcagaacgtgtatgtgaaaaccttttttggcgatgcgccgctgtgcgtggatggcagcctgaacagcgattttattgcgagc
gattggccggtgtgcgcgagctataacgaactgcaggaatttatggcgcagtttcagaacggcgaatttcgctttccgaccgaaca
tgaagtgctggcgatttataaaacccagcagcagcgcctgcataacgatgatccggattattatgatctgagcgatagcaacgtgg
gctttaaacattggcatccgagcagcaacgtggattgcattgaaggcattagcggcccgggcggccagggcgtgtgggaatgga
ccagcaccgtgtttgatagccatgaaggctttaccgtgaacgaactgtatccggaatatagcgcggatttttttgatggcaaacataa
cgtggtgctgggcggcagctgggcgaccgcgccgcgcctgagcggccgccgcagctttcgcaactggtatcagcgcagctatt
gctttgcgtggattggcgcgcgcatggtgcgcaaa
SEQ ID NO: 27
   Type: Protein Q7RX33 F358~L876
   Name: NCEGT1-tr
   Source: *Neurospora crassa* 
FSLIPSVYARSALPTLDDWEALWATWDVVTRQMLPQEELLEKPIKLRNACIFY
LGHIPTFLDIQLTKTTKQAPSEPAHFCKIFERGIDPDVDNPELCHAHSEIPDEWPPVEE
ILTYQETVRSRLRGLYAHGIANIPRNVGRAIWVGFEHELMHIETLLYMMLQSDKTLI
PTHIPRPDFDKLARKAESERVPNQWFKIPAQEITIGLDDPEDGSDINKHYGWDNEKP
PRRVQVAAFQAQGRPITNEEYAQYLLEKNIDKLPASWARLDNENISNGTTNSVSGH
HSNRTSKQQLPSSFLEKTAVRTVYGLVPLKHALDWPVFASYDELAGCAAYMGGRI
PTFEETRSIYAYADALKKKKEAERQLGRTVPAVNAHLTNNGVEITPPSSPSSETPAES
 
SSPSDSNTTLITTEDLFSDLDGANVGFHNWHPMPITSKGNTLVGQGELGGVWEWTS
SVLRKWEGFEPMELYPGYTADFFDEKHNIVLGGSWATHPRIAGRKSFVNWYQRNY
PYAWVGARVVRDL
SEQ ID NO: 28
   Type: DNA
   Name: *NCEGT1-tr*
   Source: *Neurospora crassa*

tttagcctgattccgagcgtgtatgcgcgcagcgcgctgccgaccctggatgattgggaagcgctgtgggcgacctggg
atgtggtgacccgccagatgctgccgcaggaagaactgctggaaaaaccgattaaactgcgcaacgcgtgcattttttatctggg
ccatattccgacctttctggatattcagctgaccaaaaccaccaaacaggcgccgagcgaaccggcgcatttttgcaaaatttttga
acgcggcattgatccggatgtggataacccggaactgtgccatgcgcatagcgaaattccggatgaatggccgccggtggaaga
aattctgacctatcaggaaaccgtgcgcagccgcctgcgcggcctgtatgcgcatggcattgcgaacattccgcgcaacgtggg
ccgcgcgatttgggtgggctttgaacatgaactgatgcatattgaaaccctgctgtatatgatgctgcagagcgataaaaccctgat
tccgacccatattccgcgcccggattttgataaactggcgcgcaaagcggaaagcgaacgcgtgccgaaccagtggtttaaaatt
ccggcgcaggaaattaccattggcctggatgatccggaagatggcagcgatattaacaaacattatggctgggataacgaaaaac
cgccgcgccgcgtgcaggtggcggcgtttcaggcgcagggccgcccgattaccaacgaagaatatgcgcagtatctgctggaa
aaaaacattgataaactgccggcgagctgggcgcgcctggataacgaaaacattagcaacggcaccaccaacagcgtgagcg
gccatcatagcaaccgcaccagcaaacagcagctgccgagcagctttctggaaaaaaccgcggtgcgcaccgtgtatggcctg
gtgccgctgaaacatgcgctggattggccggtgtttgcgagctatgatgaactggcgggctgcgcggcgtatatgggcggccgc
attccgacctttgaagaaacccgcagcatttatgcgtatgcggatgcgctgaaaaaaaaaaaagaagcggaacgccagctgggc
cgcaccgtgccggcggtgaacgcgcatctgaccaacaacggcgtggaaattaccccgccgagcagcccgagcagcgaaacc
ccggcggaaagcagcagcccgagcgatagcaacaccaccctgattaccaccgaagatctgtttagcgatctggatggcgcgaa
cgtgggctttcataactggcatccgatgccgattaccagcaaaggcaacaccctggtgggccagggcgaactgggcggcgtgt
gggaatggaccagcagcgtgctgcgcaaatgggaaggctttgaaccgatggaactgtatccgggctataccgcggatttttttgat
gaaaaacataacattgtgctgggcggcagctgggcgacccatccgcgcattgcgggccgcaaaagctttgtgaactggtatcag
cgcaactatccgtatgcgtgggtgggcgcgcgcgtggtgcgcgatctg

The amino acid sequences of EGT2 from 9 other strains and the nucleotide sequences of their encoding genes.
SEQ ID NO: 29
   Type: Protein 094431
   Name: SCrEGT2
   Source: *Schizosaccharomyces cryophilus* OY26 
MSDCMPFGHALKPYYMLDKNYVSVNNGSYGVVCASAFQRHLQLLEESEKTQ
DLQMKYRLPKLANNTLLQIAELLDTTSSNLAFCFSATQAISSILLTFPWSANDKILSL
NVAYPTCQFALDFVRNRYDVQVDTLEVEFIYDPSEFLSRVESYLVKNKPRVFIFDFIT
SMPVTQLPCKELIQLCKKYGVISVVDAAHGIGFCPLSLSSLDPDFLYTNAHKWLNAP
SGTTILYVSKKYHNFIEALPISYGYHIRKQNSPPADSLGIRFLNASFMDLPKFIAIDAA
IAFRKSIGGEHKIQSYNHDIAVRGSKIIAESLGTSYFALASPIAMVNVEVPLRCIPSAD
FLEEFWQSKNTFLRFVEYQGRYYTRVGGAPFLEESDFVYVADVLKELCQK
SEQ ID NO: 30
   Type: DNA
   Name: *SCrEGT2*
   Source: *Schizosaccharomyces cryophilus* OY26 
atgagcgattgcatgccgtttggccatgcgctgaaaccgtattatatgctggataaaaactatgtgagcgtgaacaacggc
agctatggcgtggtgtgcgcgagcgcgtttcagcgccatctgcagctgctggaagaaagcgaaaaaacccaggatctgcagatg
aaatatcgcctgccgaaactggcgaacaacaccctgctgcagattgcggaactgctggataccaccagcagcaacctggcgtttt
gctttagcgcgacccaggcgattagcagcattctgctgacctttccgtggagcgcgaacgataaaattctgagcctgaacgtggc
gtatccgacctgccagtttgcgctggattttgtgcgcaaccgctatgatgtgcaggtggataccctggaagtggaatttatttatgatc
cgagcgaatttctgagccgcgtggaaagctatctggtgaaaaacaaaccgcgcgtgtttatttttgattttattaccagcatgccggt
gacccagctgccgtgcaaagaactgattcagctgtgcaaaaaatatggcgtgattagcgtggtggatgcggcgcatggcattggc
ttttgcccgctgagcctgagcagcctggatccggattttctgtataccaacgcgcataaatggctgaacgcgccgagcggcacca
ccattctgtatgtgagcaaaaaatatcataactttattgaagcgctgccgattagctatggctatcatattcgcaaacagaacagccc
gccggcggatagcctgggcattcgctttctgaacgcgagctttatggatctgccgaaatttattgcgattgatgcggcgattgcgttt
cgcaaaagcattggcggcgaacataaaattcagagctataaccatgatattgcggtgcgcggcagcaaaattattgcggaaagcc
tgggcaccagctattttgcgctggcgagcccgattgcgatggtgaacgtggaagtgccgctgcgctgcattccgagcgcggattt
tctggaagaattttggcagagcaaaaacacctttctgcgctttgtggaatatcagggccgctattatacccgcgtgggcggcgcgc
cgtttctggaagaaagcgattttgtgtatgtggcggatgtgctgaaagaactgtgccagaaa
SEQ ID NO: 31
   Type: Protein XP_013025596.1
   Name: SOcEGT2
   Source: *Schizosaccharomyces octosporus* yFS286 
MSQEVPFGHALTRHFMLDENYVSVNNASCGVVCVPAFQQHIRLLEESEKVQD
LQTRYRLPKLAEQTLNQIAELLDTSPSNLAFCFSATQAISSILLTFPWTPNDRILTLNL
AYPTCQFAVDFVRDRHGIQVDTIDIDLAYNRLELLSQIESYLEKNKPRVFVFDFVTS
MPVVQLPCKEIVQICKKYNVISVIDAAHGIGLCPLSLSSLDPDFLYTNAHKWLNAPS
GTTILYVSEKYHDSIEALPISYGYHIRKENSPPADALNVRFLYASSTDLPKYIAIKDAI
AFRKSIGGEHKIQSYNYQIAIKGAKIIAEALGTSYFALAPPIAMVNVEIPLRCIPSSSFL
EEFWQKKNTFLRFVKYHGKIYTRIAGAPFLEESDFLYTVDVLKELCQFSHES
SEQ ID NO: 32
   Type: DNA
   Name: *SOcEGT2*
   Source: *Schizosaccharomyces octosporus* yFS286 
atgagccaggaagtgccgtttggccatgcgctgacccgccattttatgctggatgaaaactatgtgagcgtgaacaacgc
gagctgcggcgtggtgtgcgtgccggcgtttcagcagcatattcgcctgctggaagaaagcgaaaaagtgcaggatctgcagac
ccgctatcgcctgccgaaactggcggaacagaccctgaaccagattgcggaactgctggataccagcccgagcaacctggcgt
tttgctttagcgcgacccaggcgattagcagcattctgctgacctttccgtggaccccgaacgatcgcattctgaccctgaacctgg
cgtatccgacctgccagtttgcggtggattttgtgcgcgatcgccatggcattcaggtggataccattgatattgatctggcgtataa
ccgcctggaactgctgagccagattgaaagctatctggaaaaaaacaaaccgcgcgtgtttgtgtttgattttgtgaccagcatgcc
ggtggtgcagctgccgtgcaaagaaattgtgcagatttgcaaaaaatataacgtgattagcgtgattgatgcggcgcatggcattg
gcctgtgcccgctgagcctgagcagcctggatccggattttctgtataccaacgcgcataaatggctgaacgcgccgagcggca
ccaccattctgtatgtgagcgaaaaatatcatgatagcattgaagcgctgccgattagctatggctatcatattcgcaaagaaaaca
gcccgccggcggatgcgctgaacgtgcgctttctgtatgcgagcagcaccgatctgccgaaatatattgcgattaaagatgcgatt
 
gcgtttcgcaaaagcattggcggcgaacataaaattcagagctataactatcagattgcgattaaaggcgcgaaaattattgcgga
agcgctgggcaccagctattttgcgctggcgccgccgattgcgatggtgaacgtggaaattccgctgcgctgcattccgagcagc
agctttctggaagaattttggcagaaaaaaaacacctttctgcgctttgtgaaatatcatggcaaaatttatacccgcattgcgggcg
cgccgtttctggaagaaagcgattttctgtataccgtggatgtgctgaaagaactgtgccagtttagccatgaaagc
SEQ ID NO: 33
   Type: Protein XP_013016494.1
   Name: SOsEGT2
   Source: *Schizosaccharomyces osmophilus* 
MSECIPFGRALKPYFMLDEKYVSVNNASSGVVCVPAFQRHLQLLEESEKVQD
LQTRYRLPKLAEKTMDQLAELLDTSPNNLAFCFSATQAISSILLTFPWIANDRILTLN
LAYPTCQFAVDFVQDRYDVQMDTIDIDFAYDRSELLSRVESYLAKNKPRLFIFDFVT
SMPVIQLPCKELIQLCKKYNVISVIDAAHGIGLSPLSMSSLDPDFLYTNAHKWLNAP
SGTTILYVSKKYHNSIEALPISYGYHIRKQKSPPPAPLNARFLYASSTDLPKFIAIDAAI
AFRKSIGGEHKIQNYNYDIIIKGAKIIAEALGTSYFVLAPPIAMVNVEIPLRRMPSAEF
FEYFWQSKNTFLRFVEYHGKFYTRIAGAPFLEESDFVYIAGVLKELCQNSPEN
SEQ ID NO: 34
   Type: DNA
   Name: *SOsEGT2*
   Source: *Schizosaccharomyces osmophilus* 
atgagcgaatgcattccgtttggccgcgcgctgaaaccgtattttatgctggatgaaaaatatgtgagcgtgaacaacgcg
agcagcggcgtggtgtgcgtgccggcgtttcagcgccatctgcagctgctggaagaaagcgaaaaagtgcaggatctgcagac
ccgctatcgcctgccgaaactggcggaaaaaaccatggatcagctggcggaactgctggataccagcccgaacaacctggcgt
tttgctttagcgcgacccaggcgattagcagcattctgctgacctttccgtggattgcgaacgatcgcattctgaccctgaacctgg
cgtatccgacctgccagtttgcggtggattttgtgcaggatcgctatgatgtgcagatggataccattgatattgattttgcgtatgatc
gcagcgaactgctgagccgcgtggaaagctatctggcgaaaaacaaaccgcgcctgtttatttttgattttgtgaccagcatgccg
gtgattcagctgccgtgcaaagaactgattcagctgtgcaaaaaatataacgtgattagcgtgattgatgcggcgcatggcattgg
cctgagcccgctgagcatgagcagcctggatccggattttctgtataccaacgcgcataaatggctgaacgcgccgagcggcac
caccattctgtatgtgagcaaaaaatatcataacagcattgaagcgctgccgattagctatggctatcatattcgcaaacagaaaag
cccgccgccggcgccgctgaacgcgcgctttctgtatgcgagcagcaccgatctgccgaaatttattgcgattgatgcggcgatt
gcgtttcgcaaaagcattggcggcgaacataaaattcagaactataactatgatattattattaaaggcgcgaaaattattgcggaa
gcgctgggcaccagctattttgtgctggcgccgccgattgcgatggtgaacgtggaaattccgctgcgccgcatgccgagcgcg
gaattttttgaatatttttggcagagcaaaaacacctttctgcgctttgtggaatatcatggcaaattttatacccgcattgcgggcgcg
ccgtttctggaagaaagcgattttgtgtatattgcgggcgtgctgaaagaactgtgccagaacagcccggaaaac
SEQ ID NO: 35
   Type: Protein WBW72687.1
   Name: SJEGT2
   Source: *Schizosaccharomyces japonicus* yFS275 
MEGKDSKAVVFGHALKKEFFVDDNVVCANTGSFGTVCRQAFAATEEAVKVS
QKNTDLGFLYELPRRMRRLRSRVAEFVGAKESDIAFVGTATHAVSTILLTHPWKQG
DRLLMLSLAYPTCSFAADYVRDRYGVEIELIDVDVFDGEEFLKTVRERFEAFRPK
MFLFDLISSMPVVLTPWEKVVELCREYNVLSVVDGAHSVGLLDLNLDKVQPDFFFT
 
NTHKWLFAPSGTTILYVSEKNHSLIDPLPLSYGYGYRKEDAKPAAPFAERFRFSTYM
DSAKYLGIEGALDFRATLGEEAIRRYTNEIANKGAKIVADALGVPFTPAPFPLSMVN
VELPIKEVPALATEEFWKKHNTFVRTCEYKGKWYTRICGCIFMEESDYERVAVALK
ELCGVN
SEQ ID NO: 36
   Type: DNA
   Name: *SJEGT2*
   Source: *Schizosaccharomyces japonicus* yFS275 
atggaaggcaaagatagcaaagcggtggtgtttggccatgcgctgaaaaaagaattttttgtggatgataacgtggtgtgc
gcgaacaccggcagctttggcaccgtgtgccgccaggcgtttgcggcgaccgaagaagcggtgaaagtgagccagaaaaaca
ccgatctgggctttctgtatgaactgccgcgccgcatgcgccgcctgcgcagccgcgtggcggaatttgtgggcgcgaaagaaa
gcgatattgcgtttgtgggcaccgcgacccatgcggtgagcaccattctgctgacccatccgtggaaacagggcgatcgcctgct
gatgctgagcctggcgtatccgacctgcagctttgcggcggattatgtgcgcgatcgctatggcgtggaaattgaactgattgatgt
ggatgtggattttgatggcgaagaatttctgaaaaccgtgcgcgaacgctttgaagcgtttcgcccgaaaatgtttctgtttgatctga
ttagcagcatgccggtggtgctgaccccgtgggaaaaagtggtggaactgtgccgcgaatataacgtgctgagcgtggtggatg
gcgcgcatagcgtgggcctgctggatctgaacctggataaagtgcagccggatttttttaccaacacccataaatggctgtttgc
gccgagcggcaccaccattctgtatgtgagcgaaaaaaaccatagcctgattgatccgctgccgctgagctatggctatggctatc
gcaaagaagatgcgaaaccggcggcgccgtttgcggaacgctttcgctttagcacctatatggatagcgcgaaatatctgggcat
tgaaggcgcgctggattttcgcgcgaccctgggcgaagaagcgattcgccgctataccaacgaaattgcgaacaaaggcgcga
aaattgtggcggatgcgctgggcgtgccgtttaccccggcgccgtttccgctgagcatggtgaacgtggaactgccgattaaaga
agtgccggcgctggcgaccgaagaattttggaaaaaacataacacctttgtgcgcacctgcgaatataaaggcaaatggtatacc
cgcatttgcggctgcatttttatggaagaaagcgattatgaacgcgtggcggtggcgctgaaagaactgtgcggcgtgaac
SEQ ID NO: 37
   Type: Protein XP_002174984.1
   Name: MLEGT2
   Source: *Mucor lusitanicus* 
MGSLFGKSLRSQFLLEPQYTSLNHGSYGSIPRALLPVLDELRLKSEVNPDRWL
RREMFPVIEKNKRVVADLVHVDPQELVFVFNAMTGINTVARSLPLEAGDKILYFNT
AYNSVESTIKFIKNSEKLKLIRIDLAYPLDDSEVLAKMQETIERESSKGDGNIKLCFM
DAISSVPAVRFPFESAVKLVREHNILSLVDGAHAIGQIPLDLHQVDPDFFITNCHKWL
YAPRGCAVLYVPFRNQRMIHPAIINSAYADHSDPADKSVSFQDEFAWPGTADFSNF
MCIEAAVEFRKSIGGEDAIMTYCHELAAKGGEIAANILGTQVMGNPGQIAMVNVEI
PLTNTKLSDAQIIGIFIDKLLYEHNCMAPVYKHNGIWHTRLSAQVYNDESDFETVAK
AILAVCQELNKD
SEQ ID NO: 38
   Type: DNA
   Name: *MLEGT2*
   Source: *Mucor lusitanicus* 
atgggcagcctgtttggcaaaagcctgcgcagccagtttctgctggaaccgcagtataccagcctgaaccatggcagcta
tggcagcattccgcgcgcgctgctgccggtgctggatgaactgcgcctgaaaagcgaagtgaacccggatcgctggctgcgcc
gcgaaatgtttccggtgattgaaaaaaacaaacgcgtggtggcggatctggtgcatgtggatccgcaggaactggtgtttgtgttta
 
acgcgatgaccggcattaacaccgtggcgcgcagcctgccgctggaagcgggcgataaaattctgtattttaacaccgcgtataa
cagcgtggaaagcaccattaaatttattaaaaacagcgaaaaactgaaactgattcgcattgatctggcgtatccgctggatgatag
cgaagtgctggcgaaaatgcaggaaaccattgaacgcgaaagcagcaaaggcgatggcaacattaaactgtgctttatggatgc
gattagcagcgtgccggcggtgcgctttccgtttgaaagcgcggtgaaactggtgcgcgaacataacattctgagcctggtggat
ggcgcgcatgcgattggccagattccgctggatctgcatcaggtggatccggatttttttattaccaactgccataaatggctgtatg
cgccgcgcggctgcgcggtgctgtatgtgccgtttcgcaaccagcgcatgattcatccggcgattattaacagcgcgtatgcgga
tcatagcgatccggcggataaaagcgtgagctttcaggatgaatttgcgtggccgggcaccgcggattttagcaactttatgtgcat
tgaagcggcggtggaatttcgcaaaagcattggcggcgaagatgcgattatgacctattgccatgaactggcggcgaaaggcgg
cgaaattgcggcgaacattctgggcacccaggtgatgggcaacccgggccagattgcgatggtgaacgtggaaattccgctgac
caacaccaaactgagcgatgcgcagattattggcatttttattgataaactgctgtatgaacataactgcatggcgccggtgtataaa
cataacggcatttggcatacccgcctgagcgcgcaggtgtataacgatgaaagcgattttgaaaccgtggcgaaagcgattctgg
cggtgtgccaggaactgaacaaagat
SEQ ID NO: 39
   Type: Protein KAF1803196.1
   Name: MLCEGT2
   Source: *Mucor lusitanicus* CBS 277.49 
MTQGTTQFGKSLRSQFLLEPQYTSLNHGSYGSIPRALLPVLDELRLKSEVNPDR
WLRREMFPVIEKNKRVVADLVHVDPQELVFVFNAMTGINTVARSLPLEAGDKILYF
NTAYNSVESTIKFIKNSEKLKLIRIDLAYPLDDSEVLAKMQETIERESSKGDGNIKLC
FMDAISSVPAVRFPFESAVKLVREHNILSLVDGAHAIGQIPLDLHQVDPDFFITNCHK
WLYAPRGCAVLYVPFRNQRMIHPAIINSAYADHSDPADKSVSFQDEFAWPGTADFS
NFMCIEAAVEFRKSIGGEDAIMTYCHELAAKGGEIAANILGTQVMGNPGQIAMVNV
EIPLTNTKLSDAQIIGIFIDKLLYEHNCMAPVYKHNGIWHTRLSAQVYNDESDFETV
AKAILAVCQELNKD
SEQ ID NO: 40
   Type: DNA
   Name: *MLCEGT2*
   Source: *Mucor lusitanicus* CBS 277.49 
atgacccagggcaccacccagtttggcaaaagcctgcgcagccagtttctgctggaaccgcagtataccagcctgaacc
atggcagctatggcagcattccgcgcgcgctgctgccggtgctggatgaactgcgcctgaaaagcgaagtgaacccggatcgct
ggctgcgccgcgaaatgtttccggtgattgaaaaaaacaaacgcgtggtggcggatctggtgcatgtggatccgcaggaactgg
tgtttgtgtttaacgcgatgaccggcattaacaccgtggcgcgcagcctgccgctggaagcgggcgataaaattctgtattttaaca
ccgcgtataacagcgtggaaagcaccattaaatttattaaaaacagcgaaaaactgaaactgattcgcattgatctggcgtatccgc
tggatgatagcgaagtgctggcgaaaatgcaggaaaccattgaacgcgaaagcagcaaaggcgatggcaacattaaactgtgct
ttatggatgcgattagcagcgtgccggcggtgcgctttccgtttgaaagcgcggtgaaactggtgcgcgaacataacattctgagc
ctggtggatggcgcgcatgcgattggccagattccgctggatctgcatcaggtggatccggatttttttattaccaactgccataaat
ggctgtatgcgccgcgcggctgcgcggtgctgtatgtgccgtttcgcaaccagcgcatgattcatccggcgattattaacagcgc
gtatgcggatcatagcgatccggcggataaaagcgtgagctttcaggatgaatttgcgtggccgggcaccgcggattttagcaac
tttatgtgcattgaagcggcggtggaatttcgcaaaagcattggcggcgaagatgcgattatgacctattgccatgaactggcggc
gaaaggcggcgaaattgcggcgaacattctgggcacccaggtgatgggcaacccgggccagattcgcgatggtgaacgtggaa
attccgctgaccaacaccaaactgagcgatgcgcagattattggcatttttattgataaactgctgtatgaacataactgcatggcgc
cggtgtataaacataacggcatttggcatacccgcctgagcgcgcaggtgtataacgatgaaagcgattttgaaaccgtggcgaa
 agcgattctggcggtgtgccaggaactgaacaaagat
SEQ ID NO: 41
   Type: Protein OAD08959.1
   Name: MCEGT2
   Source: *Mucor circinelloides* 1006PhL 
MTQGTAQFGRNLRSQFLLEPQYTSLNHGSYGSIPRALLPVLEDLRLKSEVNPD
RWLRREMFPVIERNKKVVADLVHVDPQELVFVFNAMTGINTVARSLPLEAGDKVI
YFNTAYNSVESTIKFIKNSEKLKLIRIDLAYPLDDNQVLTILQETIERENSRGDGTIKL
CFMDAISSVPAVRFPFESAVKLVREHNILSLVDGAHAIGQIPLDLHQVDPDFFITNCH
KWLYAPRGCAILYVPFRNQRLIHPAIINSAYADHSDPADKSVSFQDEFAWPGTADFS
NFMCIEAAVEFRKSLGGEDAIMAYCHDLAIKGGAIAAEILGTKVMENTGQIAMVNV
EIPLGNTELSDAQIIGIFIDKLLYEHNCMAPVYKHNNIWYTRLSAQVYNDESDFETV
AKAILAVCQELDRD
SEQ ID NO: 42
   Type: DNA
   Name: *MCEGT2*
   Source: *Mucor circinelloides* 1006PhL 
atgacccagggcaccgcgcagtttggccgcaacctgcgcagccagtttctgctggaaccgcagtataccagcctgaacc
atggcagctatggcagcattccgcgcgcgctgctgccggtgctggaagatctgcgcctgaaaagcgaagtgaacccggatcgct
ggctgcgccgcgaaatgtttccggtgattgaacgcaacaaaaaagtggtggcggatctggtgcatgtggatccgcaggaactgg
tgtttgtgtttaacgcgatgaccggcattaacaccgtggcgcgcagcctgccgctggaagcgggcgataaagtgatttattttaaca
ccgcgtataacagcgtggaaagcaccattaaatttattaaaaacagcgaaaaactgaaactgattcgcattgatctggcgtatccgc
tggatgataaccaggtgctgaccattctgcaggaaaccattgaacgcgaaaacagccgcggcgatggcaccattaaactgtgctt
tatggatgcgattagcagcgtgccggcggtgcgctttccgtttgaaagcgcggtgaaactggtgcgcgaacataacattctgagc
ctggtggatggcgcgcatgcgattggccagattccgctggatctgcatcaggtggatccggatttttttattaccaactgccataaat
ggctgtatgcgccgcgcggctgcgcgattctgtatgtgccgtttcgcaaccagcgcctgattcatccggcgattattaacagcgcg
tatgcggatcatagcgatccggcggataaaagcgtgagctttcaggatgaatttgcgtggccgggcaccgcggattttagcaactt
tatgtgcattgaagcggcggtggaatttcgcaaaagcctgggcggcgaagatgcgattatggcgtattgccatgatctggcgatta
aaggcggcgcgattgcggcggaaattctgggcaccaaagtgatggaaaacaccggccagattgcgatggtgaacgtggaaatt
ccgctgggcaacaccgaactgagcgatgcgcagattattggcatttttattgataaactgctgtatgaacataactgcatggcgccg
gtgtataaacataacaacatttggtatacccgcctgagcgcgcaggtgtataacgatgaaagcgattttgaaaccgtggcgaaagc
gattctggcggtgtgccaggaactggatcgcgat
SEQ ID NO: 43
   Type: Protein EPB87019.1
   Name: TEEGT2
   Source: *Thamnidium elegans* 
MNNTTAPELGRNIREQFLFEPGYISLNHGSYGSIPKMLVPVLQDLRMKAEVNP
DRWLRREMFPEIDKNRNTLANLVHADPQELVFVFNAMTGINTVARSLPLEAGDKIL
YFNTAYNSVESTIKFVKNYEQLKLVKIDLAYPMSDSEVLDLIEETIEKENQKNDGTI
KVCFMDAISSVPAVLFPFESAIKLVRKHNILSMVDGAHAIGQIPLDLHEMDPDFFITN
CHKWLYAPRGCAVLYVPSRNQRLIHPAIINSDYADHSDPSDKTNTFQKEFAWPGTS
 
DFSNFMSINSAMEFRRSLGGEEAIMKYTRDLAVKGGEVAAKILDTDIMENAEKTLT
VAMVNVRLPLKKPNVSDVEVVQVFIDKLLNEHNCMAPVYKHNDIWYTRLSAQVY
NDVTDFEYYVAKAILSVCKELES
SEQ ID NO: 44
   Type: DNA
   Name: *TEEGT2*
   Source: *Thamnidium elegans* 
atgaacaacaccaccgcgccggaactgggccgcaacattcgcgaacagtttctgtttgaaccgggctatattagcctgaa
ccatggcagctatggcagcattccgaaaatgctggtgccggtgctgcaggatctgcgcatgaaagcggaagtgaacccggatcg
ctggctgcgccgcgaaatgtttccggaaattgataaaaaccgcaacaccctggcgaacctggtgcatgcggatccgcaggaact
ggtgtttgtgtttaacgcgatgaccggcattaacaccgtggcgcgcagcctgccgctggaagcgggcgataaaattctgtattttaa
caccgcgtataacagcgtggaaagcaccattaaatttgtgaaaaactatgaacagctgaaactggtgaaaattgatctggcgtatc
cgatgagcgatagcgaagtgctggatctgattgaagaaaccattgaaaaagaaaaccagaaaaacgatggcaccattaaagtgt
gctttatggatgcgattagcagcgtgccggcggtgctgtttccgtttgaaagcgcgattaaactggtgcgcaaacataacattctga
gcatggtggatggcgcgcatgcgattggccagattccgctggatctgcatgaaatggatccggatttttttattaccaactgccataa
atggctgtatgcgccgcgcggctgcgcggtgctgtatgtgccgagccgcaaccagcgcctgattcatccggcgattattaacagc
gattatgcggatcatagcgatccgagcgataaaaccaacacctttcagaaagaatttgcgtggccgggcaccagcgattttagca
actttatgagcattaacagcgcgatggaatttcgccgcagcctgggcggcgaagaagcgattatgaaatatacccgcgatctggc
ggtgaaaggcggcgaagtggcggcgaaaattctggataccgatattatggaaaacgcggaaaaaaccctgaccgtggcgatgg
tgaacgtgcgcctgccgctgaaaaaaccgaacgtgagcgatgtggaagtggtgcaggtgtttattgataaactgctgaacgaaca
taactgcatggcgccggtgtataaacataacgatatttggtatacccgcctgagcgcgcaggtgtataacgatgtgaccgattttga
atatgtggcgaaagcgattctgagcgtgtgcaaagaactggaaagc
SEQ ID NO: 45
   Type: Protein A7UX13
   Name: NCEGT2
   Source: *Neurospora crassa* 
MVATTVELPLQQKADAAQTVTGPLPFGNSLLKEFVLDPAYRNLNHGSFGTIPS
AIQQKLRSYQTAAEARPCPFLRYQTPVLLDESRAAVANLLKVPVETVVFVANATM
GVNTVLRNIVWSADGKDEILYFDTIYGACGKTIDYVIEDKRGIVSSRCIPLIYPAEDD
DVVAAFRDAIKKSREEGKRPRLAVIDVVSSMPGVRFPFEDIVKICKEEEIISCVDGAQ
GIGMVDLKITETDPDFLISNCHKWLFTPRGCAVFYVPVRNQHLIRSTLPTSHGFVPQ
VGNRFNPLVPAGNKSAFVSNFEFVGTVDNSPFFCVKDAIKWREEVLGGEERIMEYM
TKLAREGGQKVAEILGTRVLENSTGTLIRCAMVNIALPFVVGEDPKAPVKLTEKEE
KDVEGLYEIPHEEANMAFKWMYNVLQDEFNTFVPMTFHRRRFWARLSAQVYLEM
SDFEWAGKTLKELCERVAKGEYKESA
SEQ ID NO: 46
   Type: DNA
   Name: NCEGT2
   Source: *Neurospora crassa*

atggtggcgaccaccgtggaactgccgctgcagcagaaagcggatgcggcgcagaccgtgaccggcccgctgccgtt
tggcaacagcctgctgaaagaatttgtgctggatccggcgtatcgcaacctgaaccatggcagctttggcaccattccgagcgcg

attcagcagaaactgcgcagctatcagaccgcggcggaagcgcgcccgtgcccgtttctgcgctatcagaccccggtgctgctg
gatgaaagccgcgcggcggtggcgaacctgctgaaagtgccggtggaaaccgtggtgtttgtggcgaacgcgaccatgggcgt
gaacaccgtgctgcgcaacattgtgtggagcgcggatggcaaagatgaaattctgtattttgataccatttatggcgcgtgcggca
aaaccattgattatgtgattgaagataaacgcggcattgtgagcagccgctgcattccgctgatttatccggcggaagatgatgatg
tggtggcggcgtttcgcgatgcgattaaaaaaagccgcgaagaaggcaaacgcccgcgcctggcggtgattgatgtggtgagc
agcatgccgggcgtgcgctttccgtttgaagatattgtgaaaatttgcaaagaagaagaaattattagctgcgtggatggcgcgca
gggcattggcatggtggatctgaaaattaccgaaaccgatccggattttctgattagcaactgccataaatggctgtttaccccgcg
cggctgcgcggtgttttatgtgccggtgcgcaaccagcatctgattcgcagcaccctgccgaccagccatggctttgtgccgcag
gtgggcaaccgctttaacccgctggtgccggcgggcaacaaaagcgcgtttgtgagcaactttgaatttgtgggcaccgtggata
acagcccgtttttttgcgtgaaagatgcgattaaatggcgcgaagaagtgctgggcggcgaagaacgcattatggaatatatgacc
aaactggcgcgcgaaggcggccagaaagtggcggaaattctgggcacccgcgtgctggaaaacagcaccggcaccctgattc
gctgcgcgatggtgaacattgcgctgccgtttgtggtgggcgaagatccgaaagcgccggtgaaactgaccgaaaaagaagaa
aaagatgtggaaggcctgtatgaaattccgcatgaagaagcgaacatggcgtttaaatggatgtataacgtgctgcaggatgaatt
taacacctttgtgccgatgacctttcatcgccgccgcttttgggcgcgcctgagcgcgcaggtgtatctggaaatgagcgattttga
atgggcgggcaaaaccctgaaagaactgtgcgaacgcgtggcgaaaggcgaatataaagaaagcgcg

**Table 1. Optimization results of EGT1 tr and EGT2 in 10mM histidine betaine reaction system**

| Yield (%) | SPE GT1-tr | SOcE GT1-tr | SCrE GT1-t r | SOsEG T1-tr | SJEGT 1-tr | TDEG T1-tr | PLEG T1-tr | SCoE GT1-tr | TCEG T1-tr | NCEG T1-tr |
|---|---|---|---|---|---|---|---|---|---|---|
| SPEGT2 | 7.3 | 6.7 | 6.5 | 7.2 | 6.1 | 5.0 | 4.8 | 4.9 | 5.0 | 4.6 |
| SCrEGT2 | 6.9 | 6.8 | 6.7 | 6.9 | 6.3 | 5.3 | 4.7 | 4.5 | 5.1 | 5.0 |
| SOcEGT2 | 7.1 | 6.2 | 6.5 | 6.7 | 6.5 | 5.4 | 4.6 | 4.6 | 4.7 | 5.1 |
| SOsEGT2 | 6.7 | 6.5 | 6.3 | 7.0 | 6.0 | 4.9 | 5.1 | 4.9 | 4.8 | 4.5 |
| SJEGT2 | 5.6 | 5.6 | 6.2 | 6.1 | 6.1 | 4.6 | 4.8 | 4.3 | 4.2 | 3.8 |
| MLEGT2 | 6.2 | 5.7 | 5.8 | 5.7 | 6.3 | 3.9 | 4.2 | 3.6 | 4.1 | 3.5 |
| MLCEGT2 | 4.9 | 5.5 | 5.6 | 5.4 | 5.5 | 3.6 | 3.9 | 4.3 | 3.6 | 3.7 |
| MCEGT2 | 5.0 | 4.9 | 4.6 | 5.2 | 4.6 | 4.2 | 3.9 | 3.6 | 4.1 | 3.9 |
| TEEGT2 | 5.3 | 4.3 | 4.5 | 4.4 | 4.3 | 3.8 | 4.3 | 3.5 | 3.1 | 3.5 |
| NCEGT2 | 4.8 | 4.6 | 4.3 | 4.6 | 4.5 | 3.5 | 3.7 | 3.8 | 3.3 | 2.9 |

### Example 5

### Random mutation directed evolution of SPEGT1-tr and SPEGT2

Using the AlphaFold structures of SPEGT1 and SPEGT2 provided by Uniprot, two fragments within 0.8 nanometers of the residues enriched in substrate were selected for SPEGT1-tr and SPEGT2, respectively (SPEGT1-tr: P43~N236 and L356~A436; SPEGT2: M1~V136 and L146-I290) were randomly mutated. The specific operation for random mutation is as follows:
(1) the fragment that needs to be mutated was performed a 25 µL PCR reaction by using a Agilent GeneMorph II random mutation kit (primers and annealing temperatures used are shown in Table 2). The reaction system consists of 25ng plasmids (pET-28a-SpEgt1 tr, pET-28a-SpEgt2, or positive mutants from the previous round), 2.5 µL 10xMutazyme II reaction buffer, 0.5 µL 40mM dNTP, 1.25 µL 10 µM forward primers, 1.25 µL 10 µM reverse primers, and 0.5 µL Mutazyme II DNA polymerase. The PCR protocol involves denaturation at 95 °C for 30 seconds, annealing at 30 seconds, extension at 72 °C for 1 minute, and 30 cycles. After the reaction, 1% agarose gel was used for gel recovery and purification (see Figure 3, bands 1, 3, 5 and 7 correspond to the two fragments of SPEGT1-tr and SPEGT2 respectively). SPEGT1-tr underwent 10 rounds of random mutation, with odd rounds amplifying P43~N236 and even rounds amplifying L356~A436. SPEGT2 underwent 3 rounds of random mutation, with odd rounds amplifying M1~V136 and even rounds amplifying L146-I290.
(2) A vector corresponding to the random mutation fragment was performed a 50 µL PCR reaction by using Q5 from NEB (primers and annealing temperatures used are shown in Table 1). The reaction system consists of 1ng plasmid (randomly mutated identical plasmid), 10 µ5xQ5 reaction buffer, 1 µL 10mM dNTP, 2.5 µL 10 µM forward primer, 2.5 µL 10 µM reverse primer, and 0.5 µL Q5 High Fidelity DNA Polymerase. The PCR protocol involves denaturation at 98 °C for 10 seconds, annealing at 30 seconds, extension at 72 °C for 4 minutes, and 30 cycles. After the reaction, 1% agarose gel was used for gel recovery and purification (see Figure 3, bands 2, 4, 6 and 8 correspond to the vector part corresponding to the above random mutation fragment).
(3) A 10 µL seamless ligation reaction was performed between randomly mutated fragment and vector. The reaction system consists of 125ng vector, 25ng randomly mutated fragment, and 5 µL Seamless Cloning Kit mixture (Biyuntian). The seamless ligation protocol is 50 °C for 1 hour.

The seamless ligation product was transformed using the BL21 star (DE3) sensing state of BioNTech. Specifically, 100 µL of competent cells was added 10 µL of the seamless ligation product and mixed well. The mixture was then pleaced in a ice bath for 25 minutes, heat shocked in a 40 °C water bath for 30 seconds, then ice bath for 3 minutes. Then 1ml of LB medium without antibiotics was added and incubated on a 220rpm shaker at 37 °C for 1 hour. Every 200 µL was evenly coated onto a LB solid medium plates (5 pieces in total) containing kanamycin (K+), and incubated in a 37 °C oven for 12 hours. Generally, each tablet contains 200-500 clones.

92 clones were selected from each plate and transferred to a 96 well deep well plate containing 300 µL LB medium, the remaining 4 wells were transformed with plasmids (pET-28a-SpEgt1-tr, pET-28a-SpEgt2, or positive mutants from the previous round), then cultivated on a 600rpm shaker at 37 °C for 16 hours. Inoculated at a dose of 5% (v/v) into a new 96 well deep well plate containing 500 µL TB medium, and incubated on a 600rpm shaker at 37 °C for 2 hours, then IPTG with a final concentration of 0.3mM was added to induce expression at 20 °C for 24 hours.

The bacteria in the 96 well deep well plate were centrifuged to remove the culture medium, and 50 µL of BugBuster Protein Extraction Reagent (Merck) was added at room temperature and 450rpm for 30 minutes. Then centrifuged, the supernatant was collected to obtain bacterial lysate, then performed the reaction with the following 10mM reaction system and the reaction yield was detected using the following HPLC method to obtain the best positive mutant for this round.

After 10 rounds of random mutation, the mutant SPEGT1-tr M10 (F50L-Y51F-H97N-H99N-M151L-W376C-H405L-F425L-Y435S) was obtained, and its protein and nucleotide sequences are shown in SEQ ID No. 7 and 8. After 3 rounds of random mutation, the mutant SPEGT2 M3 (S28P-Y113F-S167A) was obtained, and its protein and nucleotide sequences are shown in SEQ ID No.: 9 and 10.

**Table 2. Primers for random mutations of Schizosaccharomyces pombe ergothioneine synthetases SPEGT1 and SPEGT2**

| Primer name | Primer sequence (5 '-3') | Primer usage | amplifier enzyme | annealing temperature |
|---|---|---|---|---|
| Spept1-tr F1 | GGACAATATGTACACCGCCTGGATTC | For random mutation of P43~N236 in SPEGT1-tr | GeneMorph II Random Mutation Kit | 55°C |
| Spegt1-tr R1 | CACCAGTTTCTTACGCATCGGTTTCTC | | | |
| Spegt1-tr F2 | CGATGCGTAAGAAACTGG | For amplifying vector | Q5 | 61°C |
| Spegt1-tr R2 | AGGCGGTGTACATATTGTC | | | |
| Spept1-tr F3 | AGCCACCGGCTTTCAGCAG | For Random mutation of L356~A436 in SPEGT1-tr | GeneMorph II Random Mutation Kit | 58°C |
| Spegt1-tr R3 | CACCAGACGTGCACCAATCCAG | | | |
| Spegt1-tr F4 | GGATTGGTGCACGTCTGG | For amplifying vector | Q5 | 67°C |
| Spegt1-tr R4 | GCTGCTGAAAGCCGGTG | | | |
| Spegt2 F1 | AGATATACCATGGGCAGCAGC | For Random mutation of M1∼V136 in SPEGT2 | GeneMorph II Random Mutation Kit | 53°C |
| Spegt2 R1 | ACAGATCTTCATCGATTTCAACACC | | | |
| Spegt2 F2 | TGAAATCGATGAAGATCTGTTTCTG | For amplifying vector | Q5 | 62°C |
| Spegt2 R2 | CTGCTGCCCATGGTATATC | | | |
| Spegt2 F3 | GGTGTTGAAATCGATGAAGATCTGTTT C | For Random mutation of 1146-1290 in SPEGT2 | GeneMorph II Random Mutation Kit | 53°C |
| Spegt2 R3 | CTGCTGGATTTTTTCTTCGCCAC | | | |
| Spegt2 F4 | GCGAAGAAAAAATCCAGCAG | For amplifying vector | Q5 | 62°C |
| Spegt2 R4 | TCTTCATCGATTTCAACACCAAC | | | |

### Example 6

### Shake bottle expression of SPEGT1 and SPEGT2

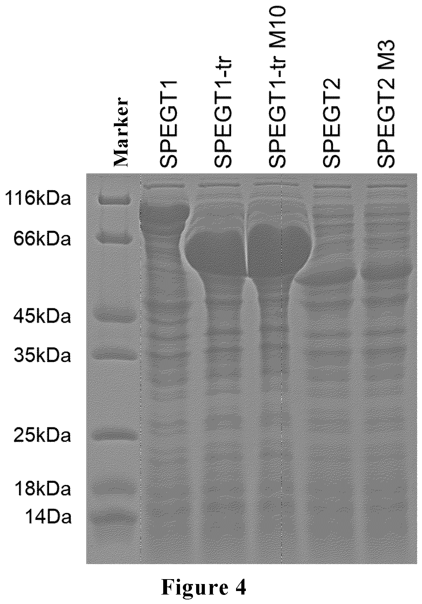

The successfully constructed engineering bacterial solution was inoculated into 1L TB liquid medium at a inoculation volume of 1% (v/v), cultured at 37 °C and 220rpm until about OD600=0.8, IPTG was added at a concentration of 0.3mM to induce expression at 20 °C for 24 hours. Then the engineered bacterias was washed with 10mM Tris at pH 8.0, centrifugated, and the bacterial cells were collectedto obtained 15.5g of bacterial cells for SPEGT1, 15.8g of bacterial cells for SPEGT1-tr, 16.5g bacterial cells for SPEGT1-tr M10; 3.8g of bacterial cells for SPEGT2, and 8.5g of bacterial cells for SPEGT2 M3. 1g of bacterial cells for each enzyme was taken out separately, 5ml of 10mM Tris (pH=8) was added and vortexed dispersion, and the bacterial solution was sonicated with a probe (50% power, 10s on 10s off; cumulative ultrasound 3000J). After the bacterial solution was lysed, centrifuged and the supernatant was subjected to 12% SDS-PAGE analysis for the expression. As shown in Figure 4, it can be seen that the expressed recombinant proteins are similar in size to the expected bands (SPEGT1 is 92 kDa, SPEGT1-tr and SPEGT1-tr M10 are 54 kDa, and SPEGT2 and SPEGT2 M3 are 47 kDa), indicating that the constructed expression vector can effectively express *Schizosaccharomyces pombe* ergothioneine synthetases and its mutants in the form of recombinant proteins. After removing the catalytic methylation domain, the expression level of SPEGT1 increased by more than 5 times.

### Example 7

### Validation of 10mM Histidine Betaine Reaction System

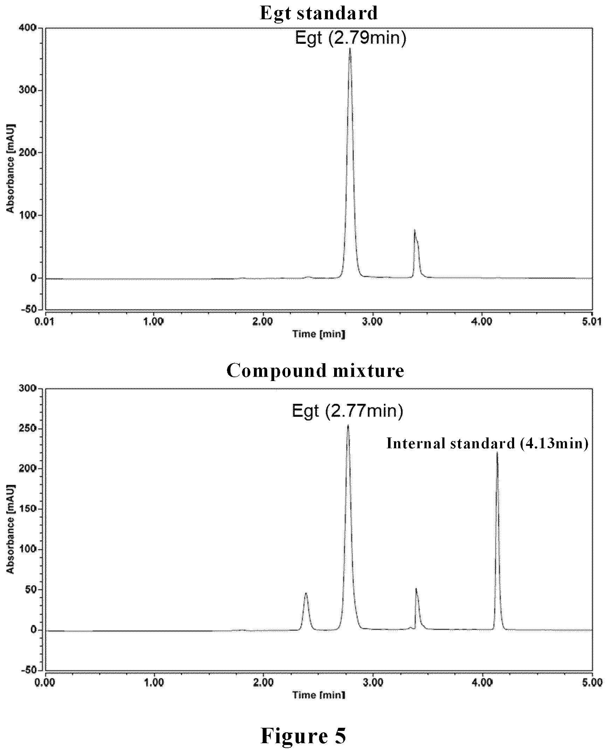

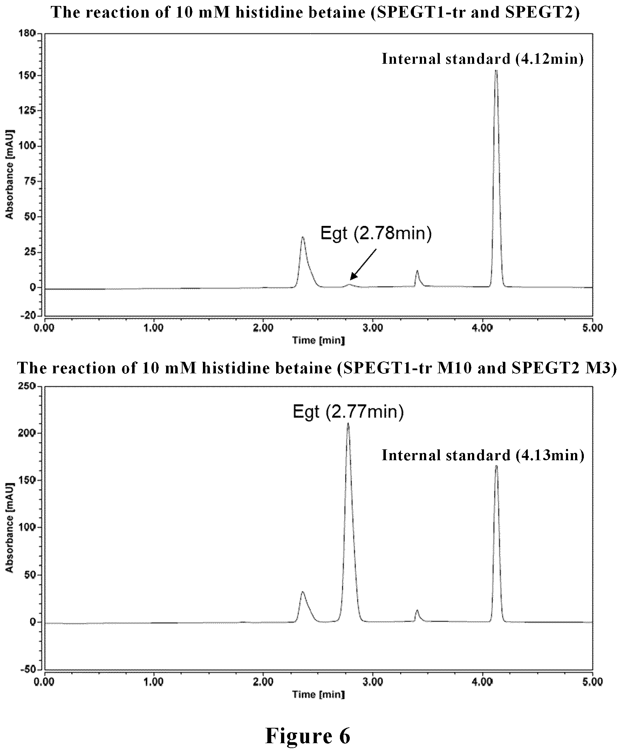

The engineering bacteria with random mutation directed evolution and shake flask expression mentioned above were validated using a 100 µL reaction system. The reaction system consists of 10mM histidine betaine, 15mM cysteine, 100mM Tris (pH 8.0), 10mM Fe²⁺, 1mM pyridoxal phosphate (PLP), 10mM beta mercaptoethanol, 1% (v/v) SPEGT1-tr (or SPEGT1-tr M10) lysate, and 1% (v/v) SPEGT2 (or SPEGT2 M3) lysate. The reaction procedure is on a 500rpm shaker at 25 °C for 2 hours. After the reaction was complete, 10mM (final concentration) of internal standard para aminobenzoic acid and 0.3mL solution of 0.1% (v/v) trifluoroacetic acid in acetonitrile were added, then centrifuged and the supernatant was collected for HPLC analysis. HPLC conditions are C18 analytical column; phase A: 50mM ammonium acetate, phase B: 100% acetonitrile; chromatographic conditions: 0%~70% phase B for 3.5 minutes, 70%~0% phase B for 1 minute, 0% phase B for 0.5 minutes; The detection wavelength is 254nm and the flow rate is 1mL/min. The spectrum of ergothioneine standard is shown in Figure 5 (Upper); the compound mixture of reaction reagent mixture in addition to 10mM ergothioneine and 10mM internal standard is shown in Figure 5 (Lower). The reaction system of 10mM histidine betaine is shown in Figure 6, where the reactions catalyzed by SPEGT1-tr and SPEGT2 are shown in Figure 6 (Upper), and the reactions catalyzed by the final mutants SPEGT1-tr M10 and SPEGT2 M3 are shown in Figure 6 (Lower). The reaction yield increased from 0.7% to 80.9%, with a 116-fold increase.

### Example 8

### 100mM Histidine Betaine Reaction System

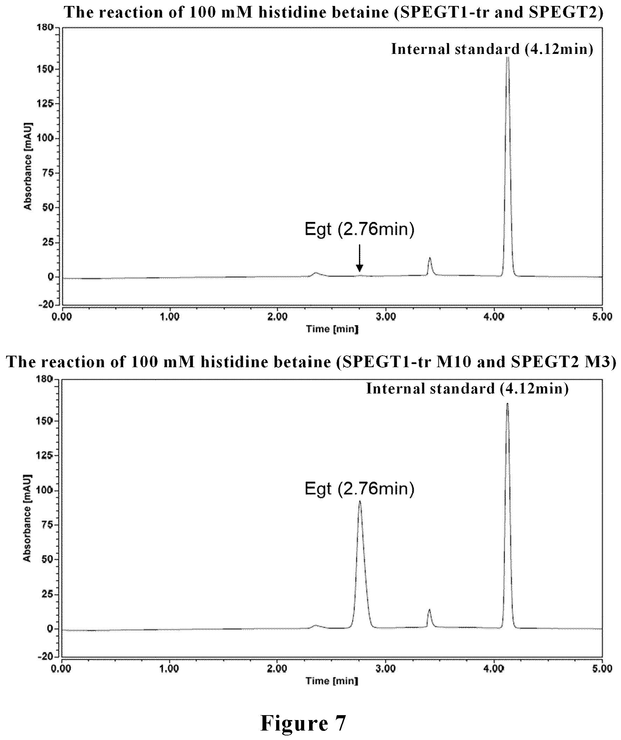

The engineering bacteria expressed in shake flasks were also validated using a high concentration 50mL reaction system. The reaction system consists of 100 mM histidine betaine, 100 mM cysteine, 100 mM Tris (pH 8.0), 10 mM Fe²⁺, 1 mM PLP, 100 mM beta mercaptoethanol, 10% (v/v) SPEGT1-tr (or SPEGT1-tr M10) lysate, and 10% (v/v) SPEGT2 (or SPEGT2 M3) lysate. The reaction procedure is on a 220rpm shaker at 25°C for 2 hours. After the reaction, internal standard para aminobenzoic acid with a final concentration of 100mM was added. Then 0.1mL of the mixture was taken out and added 0.3mL solution of 0.1% trifluoroacetic acid in acetonitrile, centrifuged and the supernatant was collected for HPLC analysis. The HPLC analysis conditions are as described above. The reaction system of 100mM histidine betaine is shown in Figure 7, where the reaction catalyzed by SPEGT1-tr and SPEGT2 are shown in Figure 7 (Upper), and the reactions catalyzed by the final mutants SPEGT1-tr M10 and SPEGT2 M3 are shown in Figure 7 (Lower). The reaction yield increased from 0.1% to 39.3%.

### Example 9

### Optimization of Fe²⁺content in 100mM histidine betaine reaction system

Fe²⁺ content in the system for catalytic synthesis of ergothioneine with the combination of evolved SPEGT1 and SPEGT2 was optimized. A high concentration 50mL reaction system was used for detection. The reaction system consists of 100 mM histidine betaine, 100 mM cysteine, 100 mM Tris (pH 8.0), 1 mM PLP, 100 mM beta mercaptoethanol, 10% (v/v) SPEGT1-tr M10 lysate, and 10% (v/v) SPEGT2 M3 lysate; wherein the Fe²⁺ content is 0mM, 1.25mM, 2.5mM, 5mM, 10mM, 20mM, and 40mM, respectively. The reaction procedure is on a 220rpm shaker at 25°C for 2 hours. After the reaction, internal standard para aminobenzoic acid with a final concentration of 100mM was added. Then 0.1mL of the mixture was taken out and added 0.3mL solution of 0.1% trifluoroacetic acid in acetonitrile, centrifuged and the supernatant was collected for HPLC analysis. The HPLC analysis conditions are as described above. The final reaction yields are shown in Table 3, where the Fe²⁺ content of 10mM was the optimal condition for the reaction system. In addition, the same experiment was conducted by replacing the metal ion Fe²⁺ with Fe³⁺ and Mn²⁺, both of which showed no activity.

**Table 3. Optimization Results of Fe²⁺ Content in 100mM Histidine Betaine Reaction System**

| Fe²⁺ content (mM) | Yield (%) |
|---|---|
| 0 | 1.4 |
| 1.25 | 11.2 |
| 2.5 | 19.4 |
| 5 | 27.5 |
| 10 | 38.7 |
| 20 | 39.6 |
| 40 | 40.2 |

### Example 10

### Optimization of the content of pyridoxal phosphate (PLP) in the 100mM histidine betaine reaction system

Content of pyridoxal phosphate (PLP) in the system for catalytic synthesis of ergothioneine with the combination of evolved SPEGT1 and SPEGT2 was optimized. A high concentration 50mL reaction system was used for detection. The reaction system consists of 100 mM histidine betaine, 100 mM cysteine, 100 mM Tris (pH 8.0), 10 mM Fe²⁺, 100 mM beta mercaptoethanol, 10% (v/v) SPEGT1-tr M10 lysate, and SPEGT2 M3 lysate; wherein, the PLP content is 0mM, 1.25mM, 2.5mM, 5mM, 10mM, 20mM, and 40mM, respectively. The reaction procedure is on a 220rpm shaker at 25°C for 2 hours. After the reaction, internal standard para aminobenzoic acid with a final concentration of 100mM was added. Then 0.1mL of the mixture was taken out and added 0.3mL of 0.1% trifluoroacetic acid in acetonitrile solution, centrifuged and the supernatant was collected for HPLC analysis. The HPLC analysis conditions are as described above. The final reaction yields are listed in Table 4, with a PLP content of 1mM being the optimal condition for the reaction system.

**Table 4. Optimization results of PLP content in 100mM histidine betaine reaction system**

| PLP content (mM) | Yield (%) |
|---|---|
| 0 | 0.4 |
| 0.125 | 5.4 |
| 0.25 | 16.6 |
| 0.5 | 28.9 |
| 1 | 39.2 |
| 2 | 39.7 |
| 4 | 41.2 |

### Example 11

### Optimization of Reducing Agent for 100mM Histidine Betaine Reaction System

The reducing agent and its content in the system for catalytic synthesis of ergothioneine with the combination of evolved SPEGT1 and SPEGT2 was optimized. A high concentration 50mL reaction system was used for detection. The reaction system consists of 100 mM histidine betaine, 100 mM Tris (pH 8.0), 10 mM Fe²⁺, 1 mM PLP, 100 mM reducing agent, 10% (v/v) SPEGT1-tr M10, and 10% (v/v) SPEGT2 M3 lysate; wherein, there are a total of 20 selected reducing agents, including 10 thiol reducing agents: β-mercaptoethanol (βME), 2-aminoethanethiol (cysteamine), dithiothreitol (DTT), dithioerythritol (DTE), glutathione (GSH), (2S) -2-amino-1,4-dimercaptobutane (DTBA), N,N'- dimethyl-N,N'-bis (mercaptoacetyl) hydrazine (DMH), meso-2,5-dimercapto-N,N,N',N'-tetramethylhexanediamine (meso DTA), bis (2-mercaptoethyl) sulfone (BMS), and 2,3-bis (mercaptomethyl) pyrazine (BMMP), three phosphorus reducing agents: trimethylphosphine (THMP), tri (2-carboxyethyl) phosphine (TCEP), tri (3-hydroxypropyl) phosphine (THPP), and 7 other common reducing agents: sodium thiosulfate (Na₂S₂O₃), sodium dithionite (Na₂S₂O₄), sodium hydrogensulfite (NaSH), sodium sulfite (Na₂SO₃), sodium ascorbate (NaVc), methanol, and sodium formate (HCO₂Na); the concentration of reducing agent is 100mM. The reaction procedure is on a 220rpm shaker at 25°C for 2 hours. After the reaction, internal standard para aminobenzoic acid with a final concentration of 100mM was added. Then 0.1mL of the mixture was taken out and added 0.3mL solution of 0.1% trifluoroacetic acid in acetonitrile, centrifuged and the supernatant was collected for HPLC analysis. The HPLC analysis conditions are as described above. The reduction effects of thiol and phosphine reducing agents are shown in Table 5, and the yields of the seven other common reducing agents mentioned above are almost zero. The optimization results demonstrate the importance of thiol/phosphine reducing agents in the reaction system.

**Table 5. Optimization results of reducing agent for 100mM histidine betaine**

| Types of reducing agents | Yield (%) |
|---|---|
| **Thiol reducing agents** | - |
| βME | 38.7 |
| Cysteine amine | 25.3 |
| DTT | 29.6 |
| DTE | 24.9 |
| GSH | 34.2 |
| DTBA | 37.4 |
| DMH | 27.3 |
| Meso-DTA | 23.4 |
| BMS | 24.6 |
| BMMP | 19.5 |
| **Phosphorus reducing agent** | - |
| THMP | 42 |
| TCEP | 47.2 |
| THPP | 46.8 |

### Example 12

### Optimization of pH condition of the 100mM histidine betaine reaction system

pH condition of the system for catalytic synthesis of ergothionein with the combination of evolved SPEGT1 and SPEGT2 was optimized. A high concentration 50mL reaction system was used for detection. The reaction system consists of 100 mM histidine betaine, 100 mM cysteine, 10 mM Fe²⁺, 1 mM PLP, 100 mM beta mercaptoethanol, 10% (v/v) SPEGT1-tr M10 lysate, and SPEGT2 M3 lysate; wherein, the pH concentrations of the reaction system were 4.5, 5, 5.5, 6, 6.5, 7, 7.5, 8, 8.5, 9, 9.5, and 10, respectively. Citric-sodium citrate buffer was used within the pH range of 4.5-5.5, K₂HPO₄-KH₂PO₄ buffer was used within the pH range of 6-7, Tris-HCl buffer was used within the pH range of 7.5-9, and glycine-sodium hydroxide buffer was used within the pH range of 9.5-10. The buffer concentrations in the system were all 100mM. The reaction procedure is on a 220rpm shaker at 25°C, for 2 hours. After the reaction, internal standard para aminobenzoic acid with a final concentration of 100mM was added. Then 0.1mL of the mixture was taken out and added 0.3mL of 0.1% trifluoroacetic acid in acetonitrile solution, centrifuged and the supernatant was collected for HPLC analysis. The HPLC analysis conditions are as described above. The final reaction yields are listed in Table 6, and the pH range of 7-9 is the optimal condition for this reaction system.

**Table 6. Optimization Results of pH Condition for 100mM Histidine Betaine Reaction System**

| pH | Yield(%) |
|---|---|
| 4.5 | 1.6 |
| 5 | 8.9 |
| 5.5 | 24.2 |
| 6 | 31.7 |
| 6.5 | 33.4 |
| 7 | 37.9 |
| 7.5 | 38.6 |
| 8 | 37.7 |
| 8.5 | 35.9 |
| 9 | 34.6 |
| 9.5 | 23.4 |
| 10 | 11.7 |

### Example 13

### Optimization of temperature condition for 100mM histidine betaine reaction system

pH condition of the system for catalytic synthesis of ergothionein with the combination of evolved SPEGT1 and SPEGT2 was optimized. A high concentration 50mL reaction system was used for detection. The reaction system consists of 100 mM histidine betaine, 100 mM Tris (pH 8.0), 100 mM cysteine, 10 mM Fe²⁺, 1 mM PLP, 100 mM beta mercaptoethanol, 10% (v/v) SPEGT1-tr M10 lysate, and SPEGT2 M3 lysate, wherein, the reaction programs were set to 10 °C, 15 °C, 20 °C, 25 °C, 30 °C, 35 °C, 40 °C, and 45 °C, respectively, and the reaction was carried out on a shaker at 220rpm for 2 hours. After the reaction, internal standard para aminobenzoic acid with a final concentration of 100mM was added. Then 0.1mL of the mixture was taken out and added 0.3mL of 0.1% trifluoroacetic acid in acetonitrile solution, centrifuged and the supernatant was collected for HPLC analysis. The HPLC analysis conditions are as described above. The final reaction yields are listed in Table 7, and the temperature range of 20-30 °C is the optimal condition for this reaction system.

**Table 7. Optimization Results of Temperature Condition for 100mM Histidine Betaine Reaction System**

| Temperature(°C) | Yield(%) |
|---|---|
| 10 | 9.2 |
| 15 | 26.4 |
| 20 | 37.1 |
| 25 | 38.9 |
| 30 | 36.4 |
| 35 | 28.4 |
| 40 | 21.6 |
| 45 | 13.2 |

All references mentioned in the invention are cited as references in this application, as if each reference were cited separately. In addition, it should be understood that after reading the above teaching content of the invention, those skilled in the art can make various modifications or modifications to the invention, and these equivalent forms also fall within the scope of the claims attached to this application.

## Claims

1. A combination of *Schizosaccharomyces pombe* ergothioneine synthetases, **characterized in that** the combination comprises: SPEGT1-tr M10, whose amino acid sequence is shown in SEQ ID NO: 7; and SPEGT2 M3, whose amino acid sequence is shown in SEQ ID NO: 9.

2. A *Schizosaccharomyces pombe* ergothioneine synthetases, **characterized in that** the methylation domain of the synthetase is truncated, and the synthetase is selected from the group consisting of: SPEGT1-tr, SPEGT1-tr M10, SOcEGT1-tr, SCrEGT1-tr, SOsEGT1-tr, SJEGT1-tr, TDEGT1-tr, PLEGT1-tr, SCoEGT1-tr, TCEGT1-tr, NCEGT1-tr, whose amino acid sequence is shown in SEQ ID NO: 5, 7, 11, 13, 15, 17, 19, 21, 23, 25, 27.

3. A ergothionein synthetase expression vector, **characterized in that** the expression vector is used to express ergothioneine synthetase EGT1-tr according to the Claim 2 and/or ergothioneine synthetase SPEGT2 M3 according to the Claim 1.

4. An expression kit, **characterized in that** the expression kit is used to express the ergothioneine synthetase EGT1-tr according to Claim 2 and/or the ergothioneine synthetase SPEGT2 M3 according to the Claim 1.

5. A recombinant strain, **characterized in that** the recombinant strain comprises an expression vector according to Claim 3, or its genome integrates polynucleotide sequences encoding the synthetases according to Claims 1 and 2.

6. A chemical enzyme coupling system for the synthesis of ergothioneine, **characterized in that** the chemical catalytic system comprises: palladium-carbon catalyzed a reduction amination dimethylation of L-histidine with formaldehyde, and undergoes a nucleophilic substitution with iodomethane to synthesize trimethylated quaternary ammonium histidine betaine;
the catalytic enzyme system comprises a combination of ergothioneine synthetases according to Claim 1 or ergothioneine synthetases according to Claim 2.

7. A method for synthesizing ergothioneine, comprising following steps: 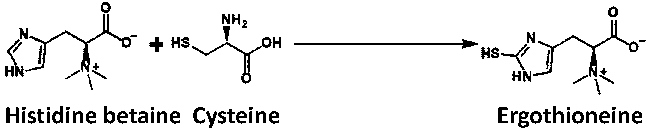 in the presence of the combination of ergothioneine synthetase according to Claim 1, or the ergothioneine synthases according to Claim 2, or the recombinant strain according to Claim 5, reacting histidine betaine with cysteine to obtain the ergothioneine.

8. The method according to Claim 7, further comprising synthesizing histidine betaine through chemical synthesis steps; preferably, the chemical synthesis step comprises: 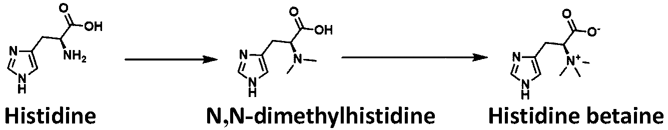
(1) under palladium-carbon catalysis, L-histidine undergoes reductive amination with formaldehyde to obtain dimethyl histidine; (2) dimethyl histidine undergoes nucleophilic substitution with iodomethane to synthesis a trimethyl substituted quaternary ammonium, histidine betaine.

9. The method according to the Claim 7, **characterized in that** the enzyme catalytic step is carried out in the presence of Fe²⁺, pyridoxal phosphate, and a reducing agent; wherein the reducing agent is a thiol reducing agent or a phosphine reducing agent used for disulfide bond reduction.

10. The method according to the Claim 7, **characterized in that** the thiol reducing agent is selected from the group consisting of beta mercaptoethanol, cysteamine, DTT, DTE, GSH, DTBA, DMH, Meso-DTA, BMS and BMMP, and/or
the phosphine reducing agent is selected from the group consisting of: THMP, TCEP and THPP.

11. The method according to the Claim 7, **characterized in that** the enzyme catalytic step is carried out in the presence of Fe²⁺, pyridoxal phosphate, and beta mercaptoethanol.

12. The method according to the Claim 7, **characterized in that** the reaction system of the enzyme catalytic step further comprises one or more features selected from the group consisting of:
(1) the Fe²⁺ content is 0-40 mM, preferably 10-40 mM;
(2) the content of pyridoxal phosphate is 0-4 mM, preferably 1-4 mM;
(3) the reducing agent content is 50-200 mM;
(4) the pH range is 4.5-10, preferably 7-9.

13. The method according to the Claim 7, **characterized in that** the enzyme catalytic step is carried out at 10-45 °C, and preferably, the enzyme catalytic step is carried out at 20-30 °C.
